# EUROPÄISCHE PATENTSCHRIFT

(11) **EP 0 824 530 B1**
(45) Veröffentlichungstag und Bekanntmachung des Hinweises auf die Patenterteilung: **02.03.2005**
(21) Anmeldenummer: 96914901.2
(22) Anmeldetag: 11.04.1996
(51) Int. Cl.: C07D 295/14, C07D 211/58, C07D 211/66, C07D 471/10, C07D 401/04, C07D 401/12, C07D 211/62, C07D 211/64, C07D 211/26, A61K 31/495, A61K 31/445

(54) **ARYLGLYCINAMIDDERIVATE, VERFAHREN ZU IHRER HERSTELLUNG UND DIESE VERBINDUNGEN ENTHALTENDE PHARMAZEUTISCHE ZUSAMMENSETZUNGEN**
ARYL GLYCINAMIDE DERIVATIVES, METHODS OF PRODUCING THESE SUBSTANCES AND PHARMACEUTICAL COMPOSITIONS CONTAINING SUCH COMPOUNDS
DERIVES D'ARYLGLYCINAMIDES, PROCEDE DE FABRICATION ET COMPOSITIONS PHARMACEUTIQUES CONTENANT CES COMPOSES

(30) Priorität: 14.04.1995 DE 19514112; 25.05.1995 DE 19519245
(43) Veröffentlichungstag der Anmeldung: 25.02.1998
(73) Patentinhaber: Boehringer Ingelheim Pharma GmbH & Co.KG, 55218 Ingelheim am Rhein (DE)
(72) Erfinder: SCHNORRENBERG, Gerd, D-55435 Gau-Algesheim (DE); DOLLINGER, Horst, D-55218 Ingelheim am Rhein (DE); ESSER, Franz, D-55218 Ingelheim am Rhein (DE); BRIEM, Hans, D-55218 Ingelheim/Rhein (DE); JUNG, Birgit, D-55270 Schwabenheim (DE); SPECK, Georg, D-55218 Ingelheim am Rhein (DE)
(74) Vertreter: Kläs, Heinz-Gerd, Dr.
(86) Internationale Anmeldenummer: PCT/EP1996/001548
(87) Internationale Veröffentlichungsnummer: WO 1996/032386

(56) Entgegenhaltungen:
- WO-A-94/01402
- WO-A-94/10146
- WO-A-95/26335
- WO-A-96/08480
- US-A- 3 518 274
- US-A- 3 862 946
- US-A- 3 906 100
- CHEMICAL ABSTRACTS, vol. 68, no. 13, 25.März 1968 Columbus, Ohio, US; abstract no. 59230g, Seite 5715; Spalte l; XP002010710 & INDIAN J. APPL. CHEM., Bd. 30, Nr. 1-2, 1967, Seite 11-13 K.J. SHAH ET AL:
- CHEMICAL ABSTRACTS, vol. 76, no. 11, 13.März 1972 Columbus, Ohio, US; abstract no. 54228t, Seite 7; Spalte l; XP002010711 & INDIAN J. PHARM., Bd. 33, Nr. 5, 1971, Seiten 86-89, PATEL B. M. ET AL:
- TETRAHEDRON LETTERS, Bd. 15, April 1967, Seiten 1387-1390, XP002010709 K. NAGARAJAN ET AL:

## Beschreibung

Die Erfindung betrifft neue Arylglycinamidderivate der allgemeinen Formel I 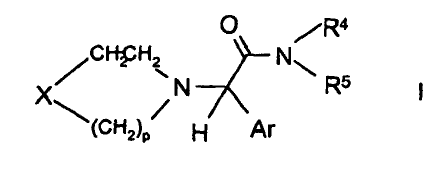 und deren pharmazeutisch annehmbare Salze, Verfahren zu ihrer Herstellung und diese Verbindungen enthaltende pharmazeutische Zusammensetzungen. Die Verbindungen sind wertvolle Neurokinin (Tachykinin)-Antagonisten.

Die in dieser Beschreibung und den Ansprüchen verwendeten Abkürzungen werden nachfolgend erklärt:
- CDI =: Carbonyldiimidazol
- DCCl =: Dicyclohexylcarbodiimid
- HOBt =: 1-Hydroxybenztriazol
- THF =: Tetrahydrofuran
- DMF =: Dimethylformamid
- RT =: Raumtemperatur
- DMAP =: 4-Dimethylaminopyridin
- TBTU =: O-Benzotriazolyl-tetramethyluroniumtetrafluoroborat

Für die Darstellung der Formeln wird eine vereinfachte Darstellung verwendet. Dabei werden in der Darstellung von Verbindungen alle CH₃-Substituenten jeweils durch einen Bindungsstrich dargestellt, so steht zum Beispiel 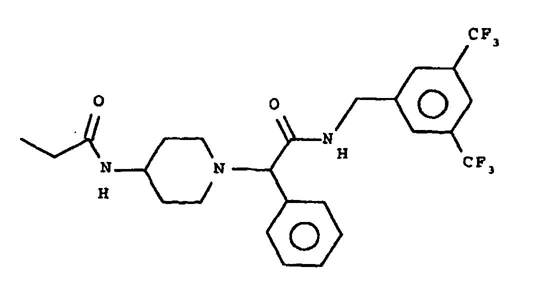 für

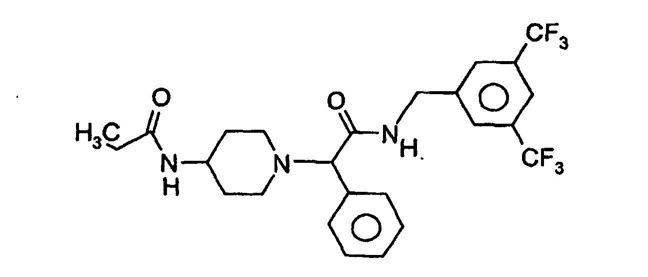

Die Erfindung betrifft neue Arylglycinamidderivate der allgemeinen Formel I 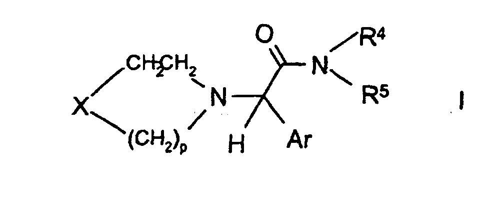 oder deren pharmazeutisch annehmbare Salze,
worin
- Ar: unsubstituiertes oder 1- bis 2-fach substituiertes Phenyl, oder unsubstituiertes Naphthyl ist, [wobei die Substituenten des Phenyl und Naphthyl unabhängig voneinander Halogen (F, Cl, Br, J), OH, Methyl, Methoxy, CF₃, OCF₃ oder Dimethylamin oder Ar durch -OCH₂O- oder -O(CH₂)₂O- substituiertes Phenyl ist; wobei diese Gruppe die Positionen 2 und 3 oder 3 und 4 des Phenyls verbinden.
- p: 2 oder 3 ist,
- X: N(CH₂)ₙR⁶ oder CR⁷R⁸ bedeutet,
worin
- n: 0, 1 oder 2 ist,
- R⁶: (C₃-C₇)Cycloalkyl oder Phenyl ist,
- R⁷: und R⁸ eine der folgenden Bedeutungen hat
a) R⁷ ist
   Phenyl, Piperidinyl, 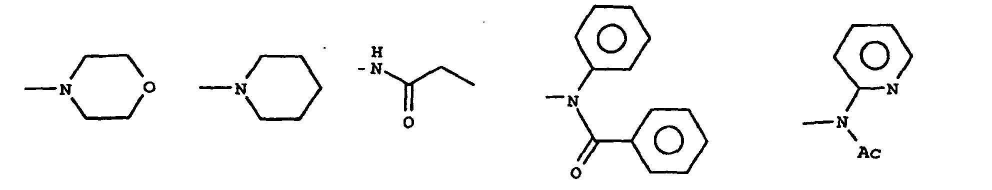 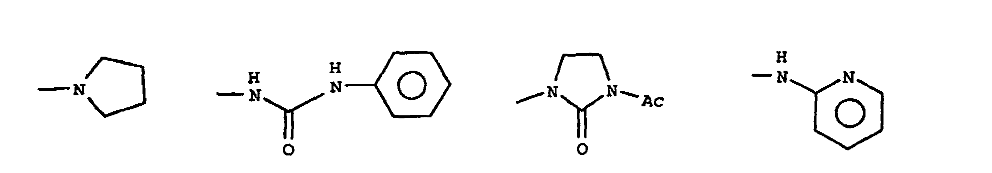 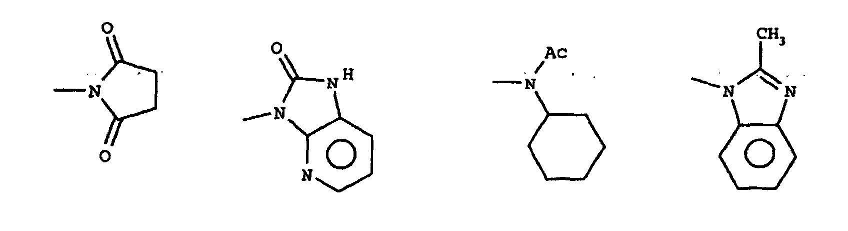 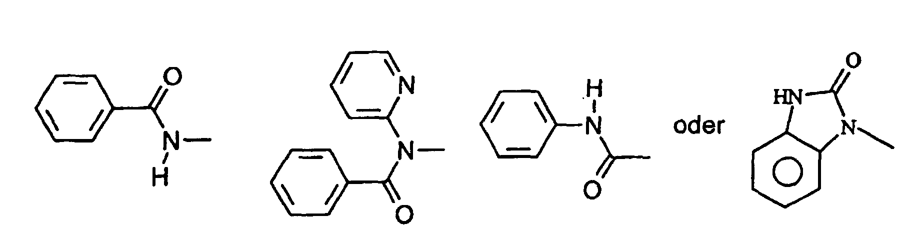 und R⁸ H, -CONH₂, -NHC(O)CH₃, -N(CH₃)C(O)CH₃, CN,
   oder
c) R⁷ und R⁸ bilden zusammen den Rest 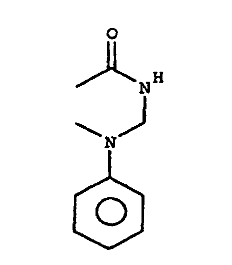
- R⁴: Phenyl(C₁-C₄)alkyl oder Naphthyl(C₁-C₄)alkyl bedeutet, worin Phenyl durch 1 bis 3 Substituenten substituiert sein kann, worin die Substituenten unabhängig voneinander Halogen (F, Cl, Br, J), (C₁-C₄)Alkyl, O-(C₁-C₄)Alkyl, CF₃, OCF₃ oder NR¹⁹R²⁰ (worin R¹⁹ und R²⁰ unabhängig voneinander H, Methyl oder Acetyl sind) sind;
und
- R⁵: H, (C₁-C₄)Alkyl, (C₃-C₆)Cycloalkyl, CH₂COOH, -CH₂C(O)NH₂, -OH oder Phenyl(C₁-C₄)alkyl bedeutet,
mit der Maßgabe dass 4-Phenyl-1-(2-phenylessigsäure-N-benzylamid)-piperidin ausgenommen ist.

Die erfindungsgemäßen Verbindungen sind wertvolle Neurokinin (Tachykinin)-Antagonisten, die sowohl Substanz P-Antagonismus, als auch Neurokinin A- bzw.

Neurokinin-B-antagonistische Eigenschaften besitzen. Sie sind nützlich zur Behandlung von und zur Vorbeugung gegenüber Neurokinin-vermittelten Krankheiten.

Die internationalen Patentanmeldungen WO 9410146 und WO 9401402 offenbaren Neurokinin Antagonisten, die allerdings keine Arylglcinamid Struktur aufweisen.

Die der Erfindung ausgenommene Verbindung 4-Phenyl-1-(2-phenytessigsäure-N-benzylamid)-piperidin wird in der Patentschrift US 3518274 als Zwischenstufe zur Darstellung von phenylsubstituierten N-(2-aminoethyl)-N-benzylamiden beschrieben.

Verbindungen der allgemeinen Formel I können Säuregruppen besitzen, hauptsächlich Carboxylgruppen, und/oder basische Gruppen wie z.B. Aminofunktionen. Verbindungen der allgemeinen Formel I können deshalb entweder als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die erfindungsgemäßen Verbindungen können als Racemate vorliegen, sie können aber auch als reine Enantiomere, d.h. in (R)- oder (S)-Form gewonnen werden.

Bevorzugt sind Verbindungen der Formel I, worin
- X: N(CH₂)ₙR⁶ ist, worin n und R⁶ ( wie oben definiert sind, insbesondere solche,
worin n 0 ist und R⁶ (C₃-C₇)Cycloalkyl, insbesondere solche Verbindungen, worin R⁶ Cyclobutyl oder Cyclohexyl ist.

Ferner sind Verbindungen der Formel I hervorzuheben, worin
- R⁷: und R⁸ eine der folgenden Bedeutungen hat
a) R⁷ ist
   Phenyl, 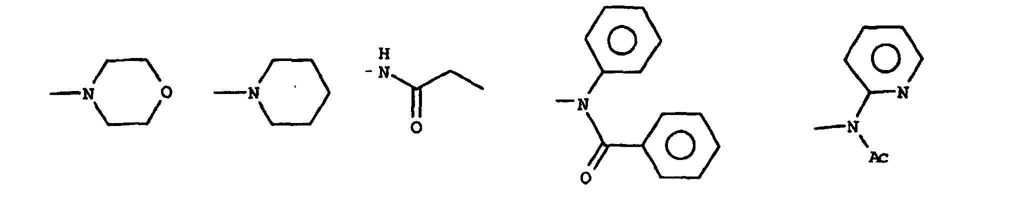 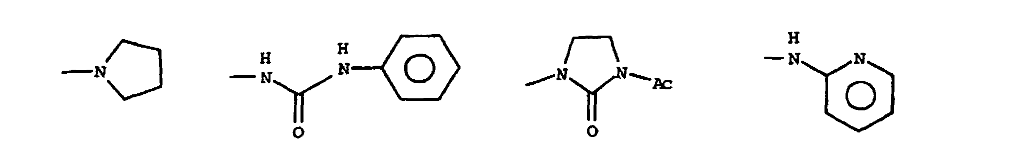 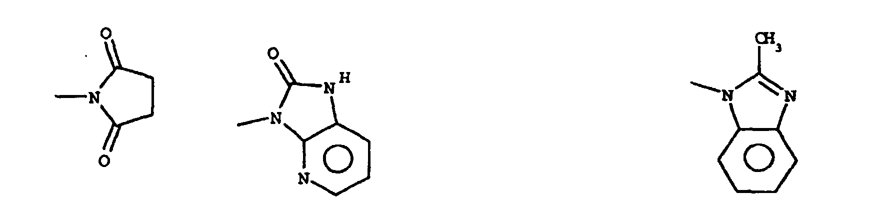 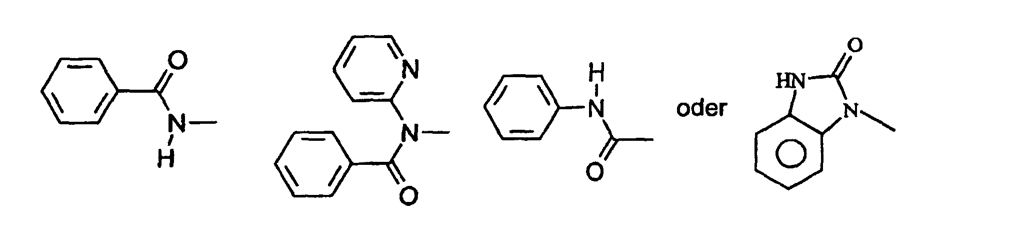 und R⁸ H, -CONH₂ oder CN ist,
   oder
c) R⁷ und R⁸ bilden zusammen den Rest

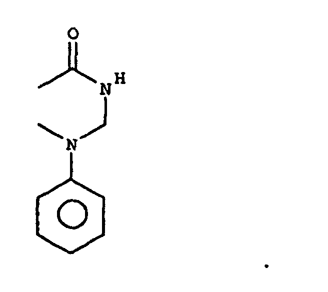

Bevorzugt sind Verbindungen, worin
- R⁷: Phenyl, 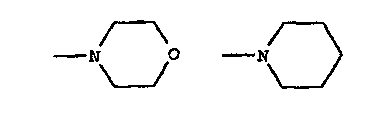 oder 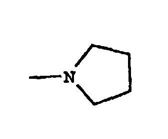 ist
und R⁸ H oder CN ist, insbesondere solche, worin R⁷ Piperidino und R⁸ H ist.

Von den oben definierten Verbindungen sind solche bevorzugt, worin
- Ar: unsubstituiertes oder 1- oder 2-fach substituiertes Phenyl, oder unsubstituiertes Naphthyl ist, [wobei die Substituenten des Phenyl unabhängig voneinander Halogen (F, Cl, Br) Methoxy oder CF₃ sind] oder Ar durch -OCH₂O- substituiertes Phenyl ist, wobei diese Gruppe die Positionen 2 und 3 oder 3 und 4 des Phenyl verbindet.

Bevorzugt sind Verbindungen, worin Ar Phenyl, 3,4-Dichlorphenyl, 3,4-Dimethoxyphenyl oder 3,4-Methylendioxyphenyl ist.

Von den oben definierten Verbindungen sind ferner solche hervorzuheben, worin
- R⁴: Phenyl(C₁-C₃)alkyl bedeutet, worin Phenyl durch 1 oder 2 Substituenten substituiert sein kann, worin die Substituenten unabhängig voneinander Halogen (F, Cl, Br, J), Methyl, Methoxy, CF₃ oder OCF₃ sind;
und
- R⁵: H, (C₁-C₃)Alkyl, CH₂COOH, -CH₂C(O)NH₂ oder Phenethyl bedeutet,
insbesondere solche Verbindungen, worin
- R⁴: 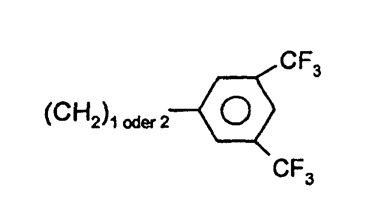 ist und R⁵ H oder CH₃.

Die folgenden Verbindungen sind bevorzugt: 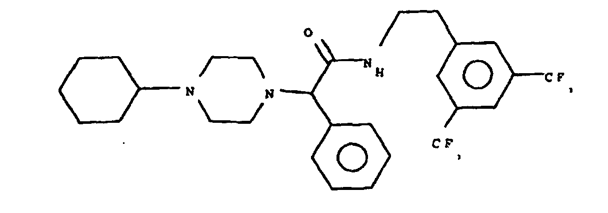 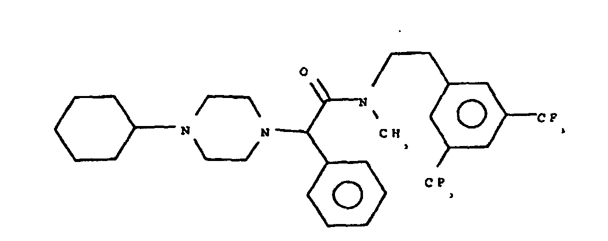 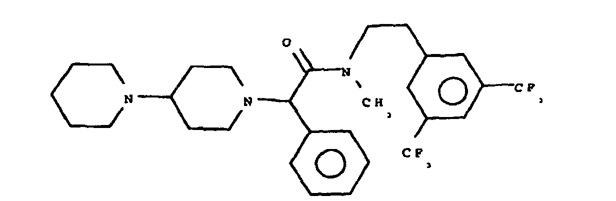 und

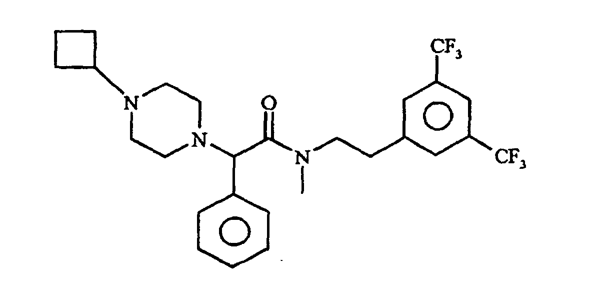

Die oben verwendete Bezeichnung Naphthyl umfaßt sowohl 1-Naphthyl als auch 2 Naphthyl.

Untersuchungsergebnisse für erfindungsgemäße Verbindungen:

Die Rezeptoraffinität zum NK₁-Rezeptor (Substanz P-Rezeptor) wird an humanen Lymphoblastoma-Zellen (IM-9) mit klonierten NK₁-Rezeptoren bestimmt, wobei die Verdrängung von ¹²⁵J-markierter Substanz P gemessen wird. Die so erhaltenen Kᵢ-Werte zeigen die Wirksamkeit der Verbindungen:

| | Kᵢ |
|---|---|
| Verbindung Beispiel 3 | 1,4 nM |
| Verbindung Beispiel 4 | 1,0 nM |
| Verbindung Beispiel 5 | 1,3 nM |
| Verbindung Beispiel 33 | 1,3 nM |
| Verbindung Beispiel 45 | 1,6 nM |
| Verbindung Beispiel 46 | 1,4 nM |
| Verbindung Beispiel 52 | 1,1 nM |
| Verbindung Beispiel 53 | 2,3 nM |
| Verbindung Beispiel 58 | 6,4 nM |
| Verbindung Beispiel 59 | 4,2 nM |
| Verbindung Beispiel 65 | 9,2 nM |
| Verbindung Beispiel 66 | 1,4 nM |
| Verbindung Beispiel 68 | 1,5 nM |
| Verbindung Beispiel 70 | 2,8 nM |
| Verbindung Beispiel 71 | 2,1 nM |
| Verbindung Beispiel 72 | 6,8 nM |
| Verbindung Beispiel 73 | 1,7 nM |
| Verbindung Beispiel 74 | 11,8 nM |
| Verbindung Beispiel 75 | 180 nM |
| Verbindung Beispiel 76 | 7,0 nM |

Die erfindungsgemässen Verbindungen sind wertvolle Neurokinin (Tachykinin)-Antagonisten, die insbesondere NK₁-Antagonismus, aber auch NK₂- und NK₃antagonistische Eigenschaften besitzen.

Die erfindungsgemäßen Verbindungen sind wertvolle Neurokinin (Tachykinin)-Antagonisten, die sowohl Substanz P-Antagonismus, als auch Neurokinin A- bzw. Neurokinin-B-antagonistische Eigenschaften besitzen. Sie sind nützlich zur Behandlung von und zur Vorbeugung gegenüber Neurokinin-vermittelten Krankheiten: Behandlung oder Vorbeugung von entzündlichen und allergischen Erkrankungen der Atemwege, wie Asthma, chronische Bronchitis, Emphysem, Rhinitis, Husten, der Augen, wie Konjunktivitis und Iritis,
der Haut, wie Dermatitis bei Kontaktekzem, Urtikaria, Psoriasis, Sonnenbrand, Insektenstiche, Neurodermitis, Juckreiz, postherpetische Schmerzen,
des Magen-Darm-Traktes, wie Magen- und Duodenalgeschwüre, Colitis ulcerosa, Morbus Crohn, Colon irritabile, M. Hirschsprung,
der Gelenke, wie rheumatoide Arthritis, reaktive Arthritis und Reiter-Syndrom;
zur Behandlung von Erkrankungen des Zentralnervensystems, wie Demenz, M. Alzheimer, Schizophrenie, Psychosen, Depression, Kopfschmerzen (z.B. Migräne oder Spannungskopfschmerzen), Epilepsie;
Behandlung von Tumoren, Kollagenosen, einer Dysfunktion der ableitenden Hamwege, von Hämorrhoiden, von Übelkeit und Erbrechen, ausgelöst z.B. durch Bestrahlung oder Zytostatikatherapie oder Bewegung und Schmerzzuständen aller Art.

Die Erfindung betrifft daher auch die Verwendung der erfindungsgemäßen Verbindungen als Heilmittel und pharmazeutische Zubereitungen, die diese Verbindungen enthalten. Bevorzugt ist die Anwendung am Menschen. Die Applikation der erfindungsgemäßen Verbindungen kann intravenös, subcutan, intramuskulär, intraperitoneal, intranasal, inhalativ, transdermal, gewünschtenfalls durch lontophorese oder literaturbekannte Enhancer gefördert, und oral erfolgen.

Zur parenteralen Applikation werden die Verbindungen der Formel I oder deren physiologisch veträglichen Salze, eventuell mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösungen oder Alkohole, z.B. Ethanol, Propandiol oder Glycerin, Zuckerlösungen wie Glucose- oder Mannit-Lösungen oder auch eine Mischung aus verschiedenen Lösungsmitteln.

Außerdem können die Verbindungen durch Implantate, z.B. aus Polylactid, Polyglycolid oder Polyhydroxybuttersäure bzw. intranasale Zubereitungen appliziert werden.

Die orale Wirksamkeit von Verbindungen der allgemeinen Formel I kann durch folgenden Standardtest gezeigt werden:
Hemmung von durch NK₁ herbeigeführte Blutdrucksenkung in anästhesierten Meerschweinchen.

Meerschweinchen (300-500 Gramm) wurden mit Pentobarbital (50 mg/kg i.p.) anästhesiert, intubiert und mechanisch beatmet mit 10 ml Raumluft pro kg Körpergewicht und einer Frequenz von 60 Atemzügen pro Minute. Die Blutdruckmessung erfolgte blutig über die Halsschlagader. Zur intravenösen Zufuhr von Substanzen wurde die Halsvene kanüliert.
Durch intravenöse Gabe von dem NK₁-Agonist [βAla⁴, Sar⁹, Met(O₂)¹¹] SP(4-11)

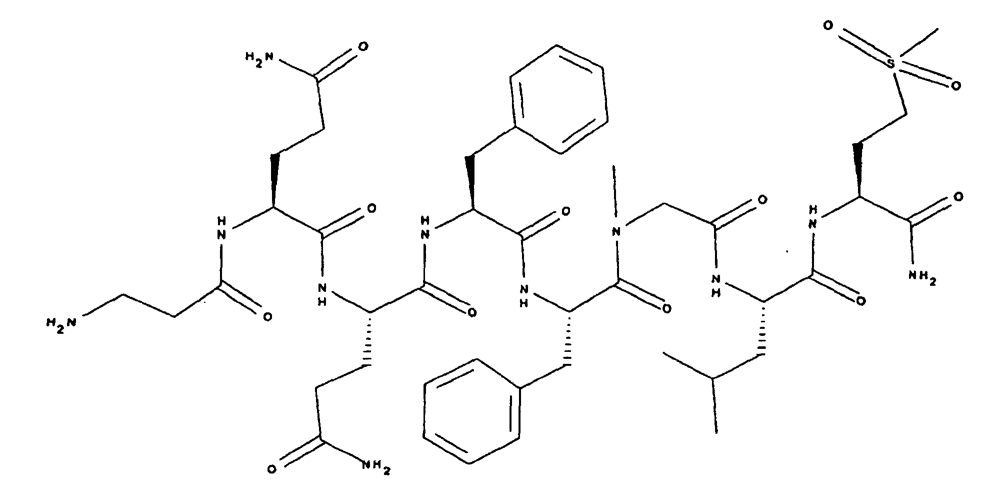

(0,2 µmol/kg) wurde eine kurzdauernde Blutdrucksenkung ausgelöst, die durch erneute Gabe des NK₁-Agonisten in Abständen von 10 Minuten wiederholt wurde.

Anschließend wurde der Neurokinin-Antagonist intraduodenal verabreicht und eine in 10 Minuten Intervallen eine Blutdrucksenkung durch den NK₁-Agonisten induziert.

Die Hemmung der durch den genannten NK₁-Agonisten verursachten Blutdrucksenkung vor und nach Behandlung mit dem Neurokinin-Antagonisten wurde ermittelt.

Die Verbindung des Beispiels 5 ergab ID₅₀ = 1,4 mg/kg. (ID₅₀ ist die Dosis, die die durch den NK₁-Agonisten verursachte Blutdrucksenkung um 50% hemmt.)

Die erfindungsgemäßen Verbindungen können nach allgemein bekannten Methoden hergestellt werden.

Die Herstellung der Verbindungen kann auf verschiedene Weise erfolgen. Die beiden gebräuchlichsten Verfahren sind im folgenden Schema dargestellt:

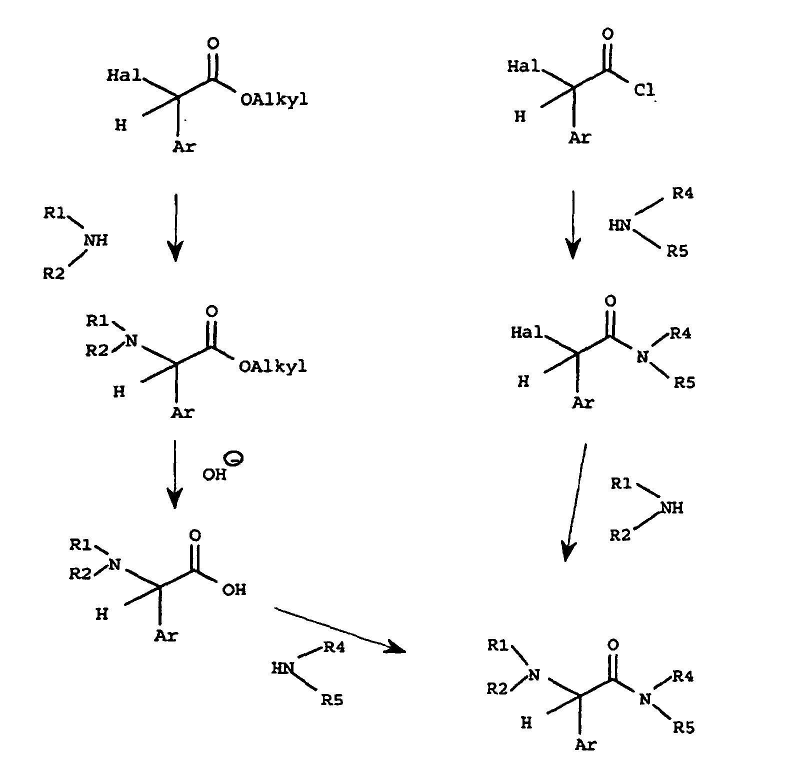

Verfahren A. Die Verknüpfung der Carbonsäure mit dem Amin HN (R⁵)R⁴ kann auf verschiedene Weise erfolgen. Übliche Methoden sind Kupplungsverfahren wie sie in der Peptidchemie angewendet werden. Dabei wird ein Kupplungsreagens wie TBTU, DCCl / HOBt, CDI, etc. in etwa äquivalenter Menge zu den Kupplungspartnern eingesetzt. Geeignete Lösungsmittel sind DMF, THF, CH₂ Cl₂, CHCl₃, Acetonitril oder andere indifferente Lösungsmittel oder deren Gemische. Der geeignete Temperaturbereich liegt zwischen -50°C und + 120°C, bevorzugt zwischen 0°C und 40°C.

Die Carbonsäure kann auch zunächst mittels SOCl₂, SO₂Cl₂, PCl₃, PCl₅ oder PBr₃ oder deren Gemischen nach bekannten Verfahren in das entsprechende Säurehalogenid überführt werden, das anschließend in einem indifferenten Lösungsmittel wie z.B. CH₂Cl₂, THF oder Dioxan bei Temperaturen zwischen - 50°C und +100°C, typischerweise bei 0° bis 20°C mit dem Amin HN (R⁵)R⁴ umgesetzt wird.
Eine weitere Alternative besteht darin, die Carbonsäure nach bekannten Methoden zunächst in den Alkylester, üblicherweise den Methylester zu überführen, der dann in einem indifferenten Lösungsmittel wie z.B. DMF, Dioxan oder THF mit dem Amin HN(R⁵) R⁴ zur Reaktion gebracht wird. Die Reaktionstemperaturen liegen zwischen 20°C und 150°C, typischerweise zwischen 50°C und 120°C. Die Reaktion kann auch in einem Druckbehälter durchgeführt werden.

Verfahren B. Hierbei wird das nach bekannten Vorgehensweisen erhaltene α-Halogen-arylacetamidderivat mit dem Amin R¹(R²)NH unter Abspaltung von Halogen-wasserstoff zur Reaktion gebracht. Zum Abfangen des abgespaltenen (oder auch überschüssigen) Halogenwasserstoffs verwendet man anorganische Basen wie z.B. K₂CO₃, NaHCO₃ oder CaCO₃ oder organische Basen wie z.B. Triethylamin, Hünig-Base, Pyridin oder DMAP, oder man verwendet das Amin R¹(R²)NH im Überschuß. Dabei verwendet man DMF, THF, Dioxan oder andere indifferente Lösungsmittel. Der Temperaturbereich für die Reaktion liegt bei 0° - 100°C, typischerweise zwischen 10° und 80°C.

Verfahren C. Die erfindungsgemäßen Verbindungen in denen R⁵ nicht H ist, können auch wie folgt hergestellt werden: Zunächst synthetisiert man z.B. nach Verfahren A oder B die entsprechende Verbindung in der R⁵ H ist. Anschließend führt man wie folgt eine N-Alkylierung durch, um so Alkyl, Cycloalkyl oder CH₂COOH einzuführen. Die erfindungsgemäße Verbindung worin R⁵ H ist wird mit einer äquivalenten Menge NaH, NaNH₂, KOH, NaOCH₃ oder einer anderen starken Base deprotoniert. Dabei verwendet man wasserfreie, indifferente Lösungsmittel wie z.B. THF, Dioxan oder Diethylether. Anschließend gibt man das entsprechende Alkylierungsmittel in Form des entsprechenden Halogenids, Tosylats oder Mesylats langsam zu. Die Umsetzung wird im Temperaturbereich - 50°C bis +100°C durchgeführt, typischerweise zwischen 0°C und +50°C. Das Verfahren ist in Beispiel 33 detailliert beschrieben.

### Beispiel 1:

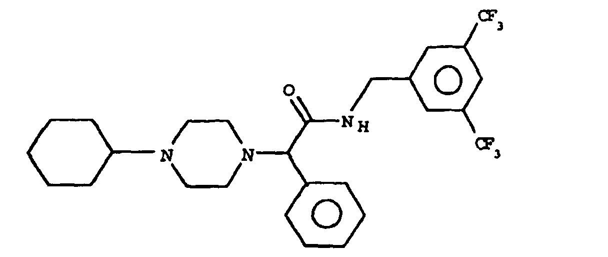

1. Stufe: 2,2 g 1-Cyclohexylpiperazin wurden in 150 ml wasserfreiem DMF gelöst, mit 2 g K₂CO₃ versetzt, 20 min. bei Raumtemperatur gerührt und dann auf 5°C abgekühlt. Es wurden 2,7 g (R,S)- α-Bromphenylessigsäuremethylester zugegeben und die Suspension über Nacht bei Raumtemperatur gerührt. Der Niederschlag wurde abfiltriert und das Filtrat eingeengt. Der Rückstand wurde in Essigester aufgenommen, 2 x mit 10%iger KHCO₃-Lösung und 1 x mit gesättigter NaCl-Lösung extrahiert. Die organische Phase wurde über Na₂SO₄ getrocknet, filtriert und eingeengt, wobei 3,7 g (R,S)-1-Cyclohexyl-4-(2-phenylessigsäuremethylester)-piperazin als gelbes Öl erhalten wurden. Ausbeute: ca. 100%.
2. Stufe: 2,3 g des Produkts aus der 1. Stufe wurden in 10 ml Methanol gelöst, mit 14 ml 1 N NaOH versetzt und die entstehende Emulsion über Nacht bei Raumtemperatur gerührt. Die klare Reaktionslösung wurde durch Zugabe von 14 ml 1 N HCl neutralisiert, zur Trockne eingeengt, der Rückstand mit Isopropanol behandelt und der Feststoff abgesaugt. Das Filtrat wurde eingeengt und der Rückstand erneut mit Isopropanol verrieben, der Feststoff abgesaugt und mit dem zuvor erhaltenen Feststoff vereinigt. So wurden 1,6 g (R,S)-1-Cydohexyl-4-(2-phenylessigsäure)-piperazin als weisse Festsubstanz erhalten. Ausbeute: 75%.
3. Stufe: 0,6 g des Produkts aus der 2.Stufe, 0,48 g 3,5-Bis-(trifluormethyl)-benzylamin und 0,32 g HOBT wurden in 60 ml THF/CH₂Cl₂ (1 : 1) suspendiert und durch Zugabe von ca. 0,7 ml Hünig-Base auf pH 8,5 eingestellt. Es wurde mit 0,77 g TBTU versetzt und bei Raumtemperatur über Nacht gerührt. Die klare Reaktionslösung wurde unter Vakuum eingeengt, der Rückstand in CH₂Cl₂ aufgenommen und 2 x mit 10%iger, KHSO₄-Lösung,
1 x mit ges. NaCl-Lösung, 2 x mit 10%iger KHCO₃-Lösung und 1 x mit ges. NaCl-Lösung ausgeschüttelt. Die organische Phase wurde über Na₂SO₄ getrocknet, abfiltriert und eingeengt wobei Kristallisation eintrat. Es wurden 0,685 g (R,S)-1-Cyclohexyl-piperazinyl-4-[2-phenylessigsäure-N-(3,5-bis-trifluormethylbenzyl)amid] als gelbliche Festsubstanz erhalten. Ausbeute: 64 %.
Fp.: 124 - 129°C. FAB-MS: (M+H)⁺ = 528,2.

### Beispiel 2:

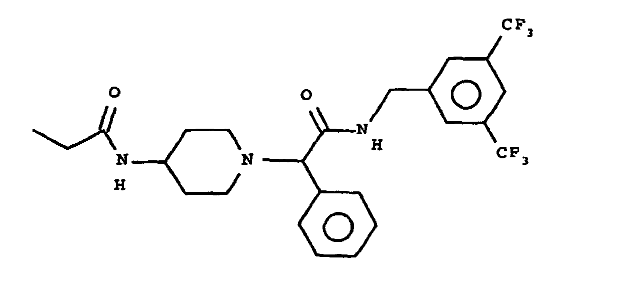

1. Stufe: 0,49 g 3,5-Bis-(trifluormethyl)-benzylamin wurde in 30 ml wasserfreiem CH₂Cl₂ gelöst, mit 0,3 ml Triethylamin versetzt, die Mischung im Eisbad gekühlt und innerhalb von 20 min. eine Lösung von 0,46g (R,S)-α-Bromphenylessigsäurechlorid in 10 ml CH₂Cl₂ zugetropft. Nach einem Wochenende bei Raumtemperatur wurde das Lösungsmittel abgezogen und der feste Rückstand mit Diethylether verrieben, abgesaugt und das Filtrat eingeengt. Dabei wurden 0,6 g α-Bromphenylessigsäure-N-(bis-trifluormethyl-benzyl)-amid als hellbeige Festsubstanz erhalten.
   Ausbeute: 43,5 %.
2. Stufe: 0,21 g 4-Propionylamino-piperidin-hydrochlorid wurden in 30 ml wasserfreiem DMF gelöst, mit 0,33 g K₂CO₃ versetzt und 30 min. bei Raumtemperatur gerührt. Zu dieser Mischung wurden innerhalb von 20 Min. eine Lösung von 0,68 g des Produkts der 1. Stufe in 10 ml DMF zugetropft und über Nacht bei Raumtemperatur gerührt. Die Suspension wurde filtriert, das Filtrat eingeengt, der erhaltene ölige Rückstand in Essigester aufgenommen, 2 x mit 10%iger KHCO₃-Lösung und 1 x mit ges. NaCl-Lösung extrahiert. Die organische Phase wurde über Na₂SO₄ getrocknet, filtriert, das Filtrtat eingeengt und der erhaltene halbfeste Rückstand mit Diethylether verrieben und abgesaugt. Dabei wurden 0,33 g (R,S)-4-Propionylamino-1-[2-phenylessigsäure-N(3,5-bistrifluormethyl-benzyl)-amid]-piperidin als weiße Festsubstanz erhalten. Ausbeute: 64 %. Fp.: 189 - 191 ° C
   FAB-MS: (M+H)⁺ = 516, 4.

### Beispiel 33

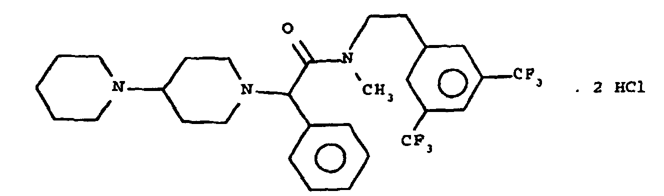

Fp.: > 240°C; FAB-MS: (M+H)⁺ = 556,4

0,3 g der Verbindung Beispiel 25 wurden durch Behandlung mit KHCO₃ in die entsprechende Base überführt und getrocknet. Diese wurde in 5ml wasserfreiem THF gelöst, mit 34 mg NaH (60 %ig in Öl) versetzt und 1,5 h bei Raumtemperatur gerührt. Dann wurden 0,1 g Methyljodid hinzugegeben und über Nacht gerührt. Die Reaktionsmischung wurde mit 2 ml THF/Wasser (1:1), dann mit 25 ml Wasser versetzt und 3x ausgeethert. Die vereinigten Etherextrakte wurden über Na₂SO₄ getrocknet und im Vakuum eingeengt wodurch 170 mg der gewünschten Verbindung als freie Base (Öl) erhalten wurden. Diese wurde durch Zugabe von etherischen HCl im Überschuß in das Dihydrochlorid überführt, das in Form gelber Kristalle anfiel.
Ausbeute: 113 mg (36 %).

Analog können die anderen Verbindungen dieser Erfindung hergestellt werden, zum Beispiel die folgenden:

### Beispiel 3

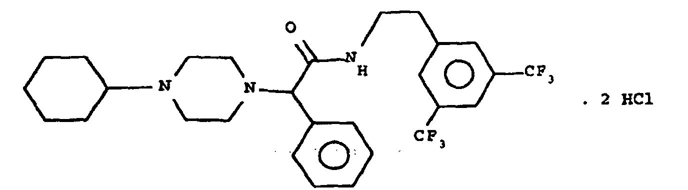

Fp: 235 - 238°C. FAB-MS: (M+H)⁺ = 542,2.

### Beispiel 4

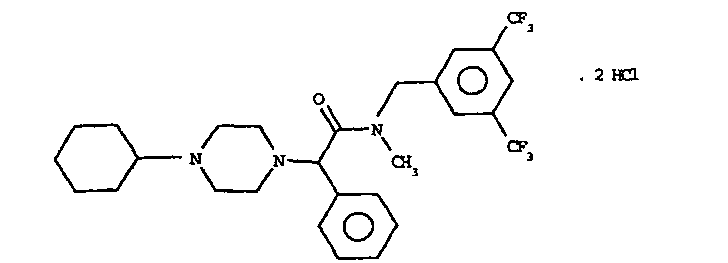

Fp: > 240°C (Zers.). FAB-MS: (M + H)⁺ = 542,3.

### Beispiel 5

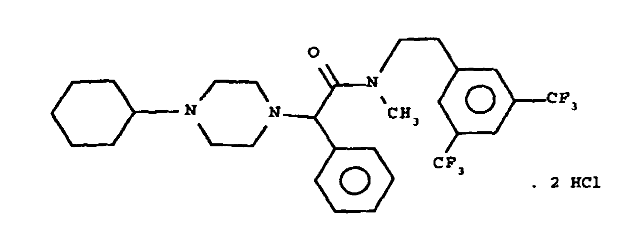

Fp.: 158 - 164°C; FAB-MS: (M+H)⁺ = 556,4.

### Beispiel 6:

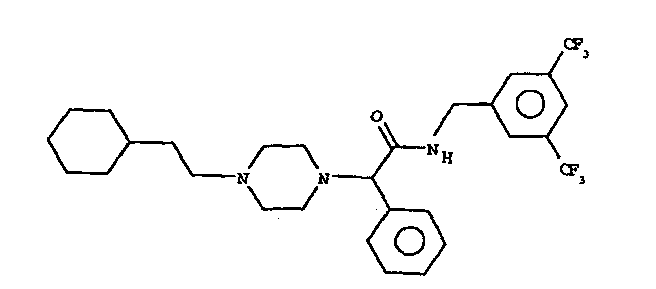

Fp.: 97 - 99°C; FAB-MS: (M+H)⁺ = 556,3.

### Beispiel 7:

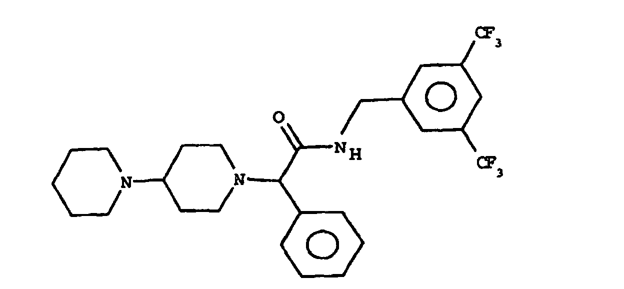

Fp: > 240° (Zers.); FAB-MS: (M+H)⁺ = 528,4.

### Beispiel 8:

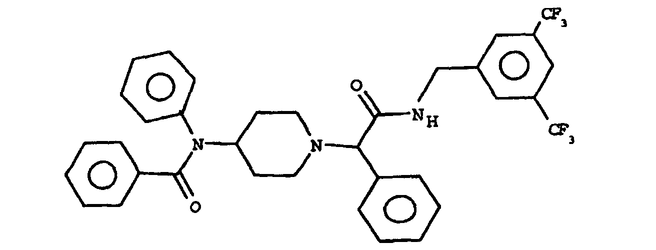

Fp.: 102 - 105° C; FAB-MS: (M+H)⁺ = 640,3.

### Beispiel 9:

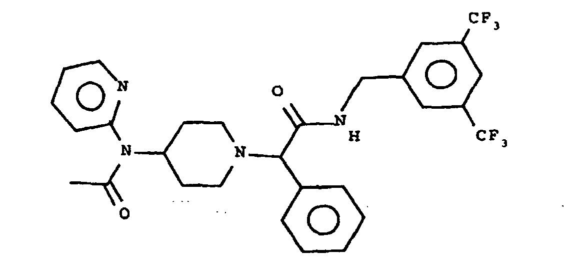

Fp.: 141-149°C; FAB-MS: (M+H)⁺ = 579,2.

### Beispiel 10:

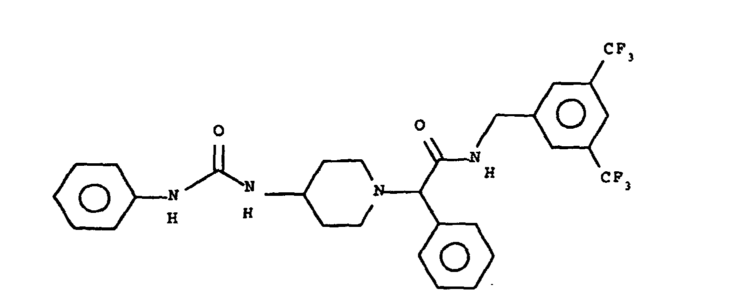

Fp: 218-223°C; FAB-MS: (M+H)⁺ = 579,3.

### Beispiel 11:

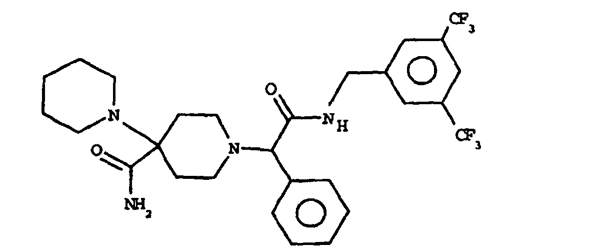

Fp.: > 220° (Zers.); FAB-MS (M+H)⁺ = 571,3

### Beispiel 12:

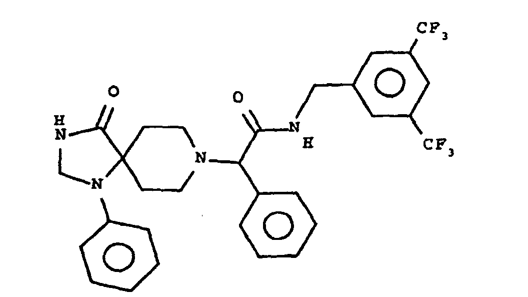

Fp.: 205-210°C; FAB-MS: (M+H)⁺ = 591,3.

### Beispiel 13:

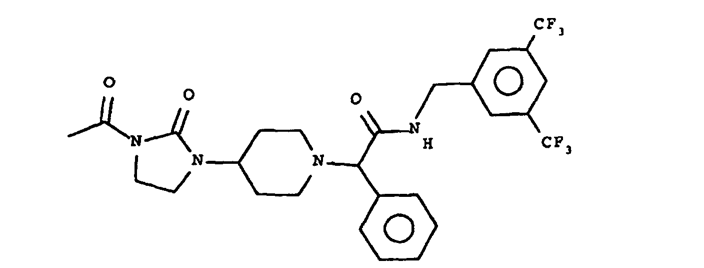

Fp.: 87 - 95°C; FAB-MS: (M+H)⁺ = 571,2

### Beispiel 14:

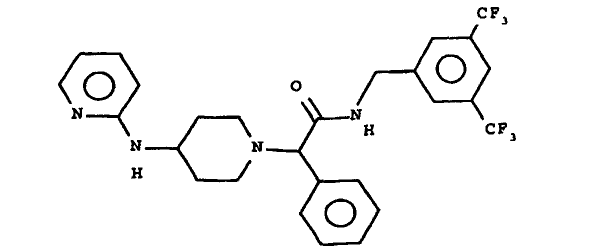

Fp.: 164-166°C; FAB-MS: (M+H)⁺ = 537,3.

### Beispiel 15:

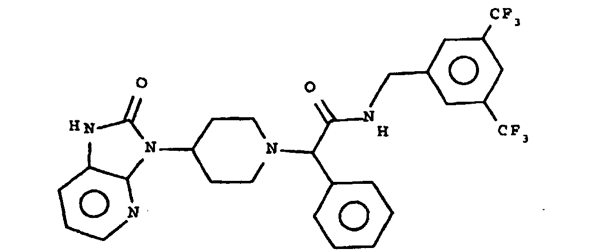

Fp.: 208 - 210°C; FAB-MS: (M+H)⁺ = 578,3.

### Beispiel 16:

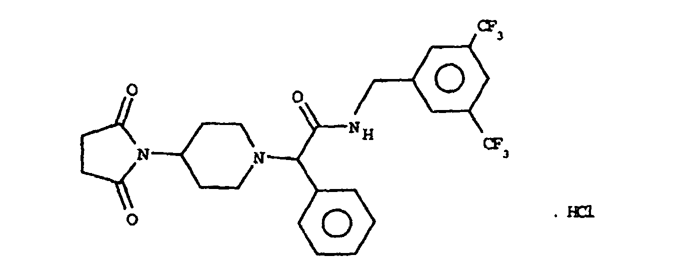

Fp.: 110-115°C; FAB-MS: (M+H)⁺ = 542,3.

### Beispiel 17:

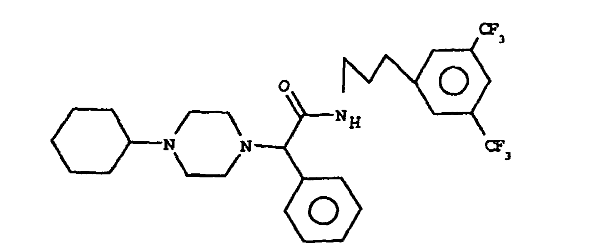

Fp.: 118 - 123 ° C; FAB-MS: (M+H)⁺ = 556,3

### Beispiel 18:

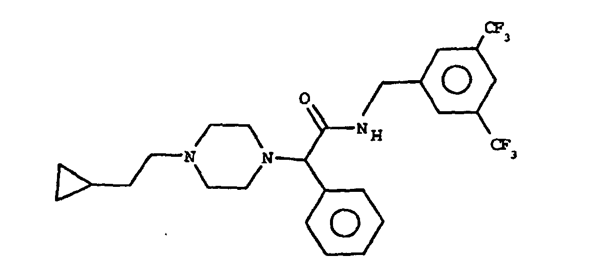

Fp: 134 - 136°C; FAB-MS: (M+H)⁺ = 514,3

### Beispiel 19:

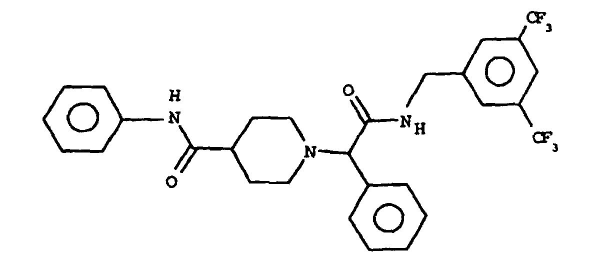

Fp: > 240° (Zers.); FAB-MS: (M+H)⁺ = 564

### Beispiel 20:

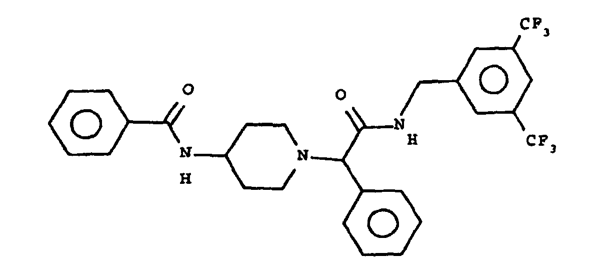

Fp.: 180 - 185°C; FAB-MS: (M+H)⁺ = 564,3

### Beispiel 21:

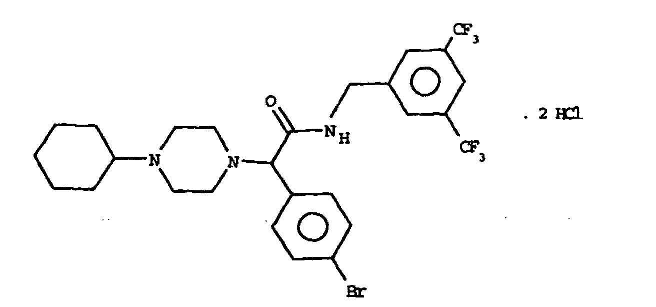

Fp: 228 - 232 °C; FAB-MS: (M + H)⁺ = 606/608

### Beispiel 22:

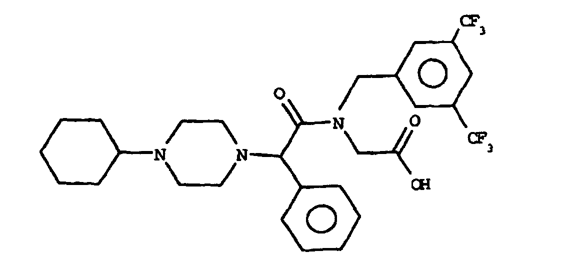

Fp: 70 - 73°C; FAB-MS: (M+H)⁺ = 586

### Beispiel 23:

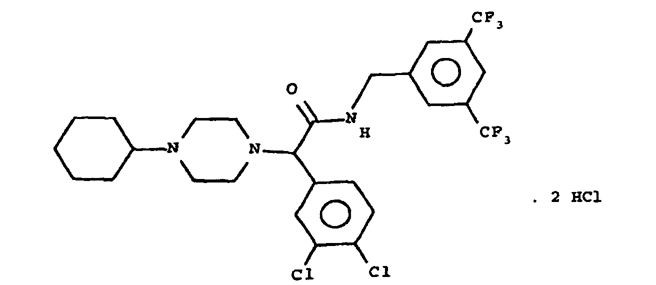

Fp: 248 - 254°C; FAB-MS: (M+H)⁺ = 596/598/600.

### Beispiel 24:

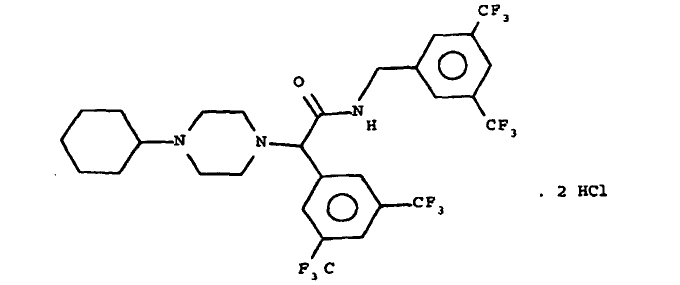

Fp.: 210°C; FAB-MS: (M+H)⁺ = 664,1

### Beispiel 25:

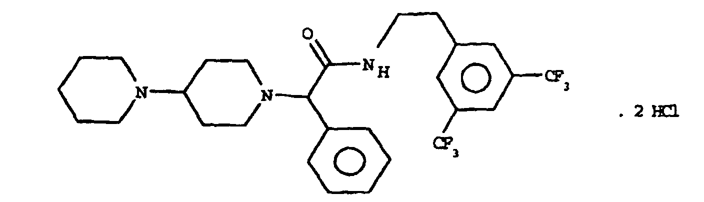

Fp.: 192 - 199°C; FAB-MS: (M+H)⁺ = 542,3

### Beispiel 26:

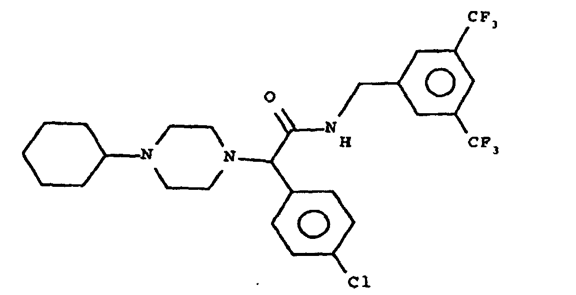

Fp.: 112 - 118°C; FAB-MS: (M+H)⁺ = 562/564

### Beispiel 27:

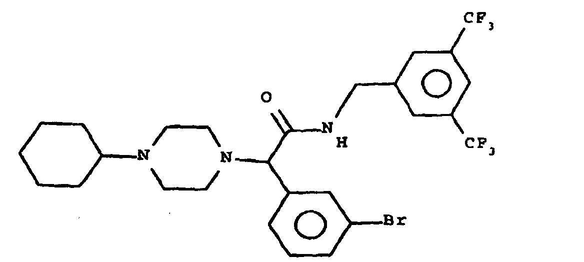

Fp: 124 - 127°C; FAB-MS: (M+H)⁺ = 606/608

### Beispiel 28:

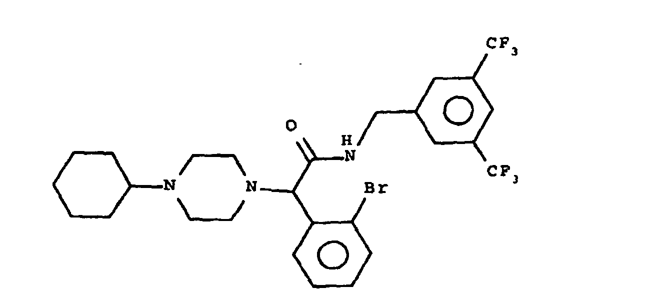

Fp.: 118 - 120°C; FAB.MS: (M+H)⁺ = 606/608.

### Beispiel 29:

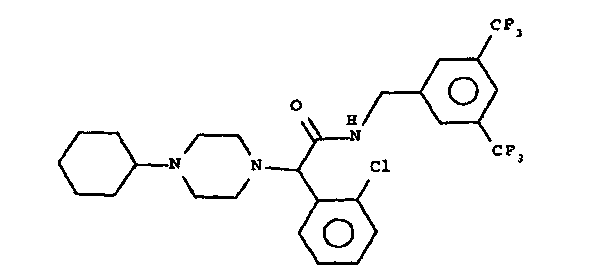

Fp.: 120 - 122°C; FAB-MS: (M+H)⁺ = 562/564

### Beispiel 30:

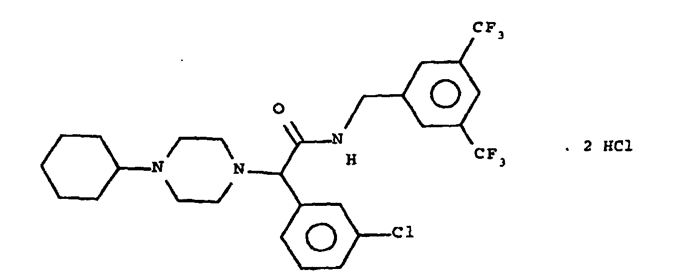

Fp: > 240°C; FAB-MS: (M+H)⁺ = 562/564

### Beispiel 31:

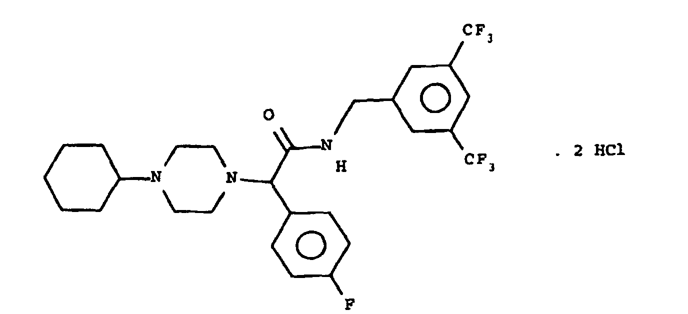

Fp.: > 240°C; FAB-MS: (M+H)⁺ = 546,3

### Beispiel 32:

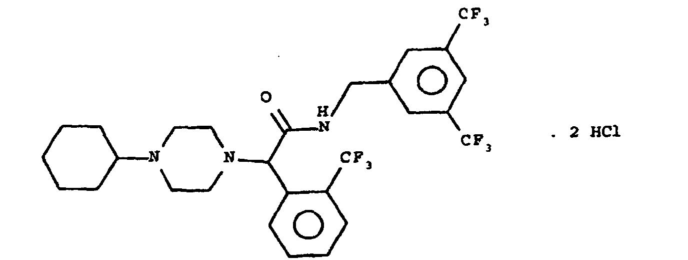

Fp.: 125- 130°C (Zers.); FAB-MS: (M+H)⁺ = 610,4

### Beispiel 33:

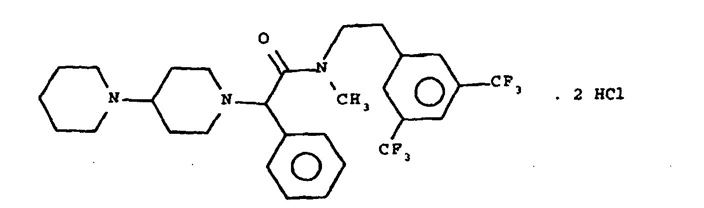

Fp.: > 240°C; FAB-MS: (M+H)⁺ = 556,4

### Beispiel 34:

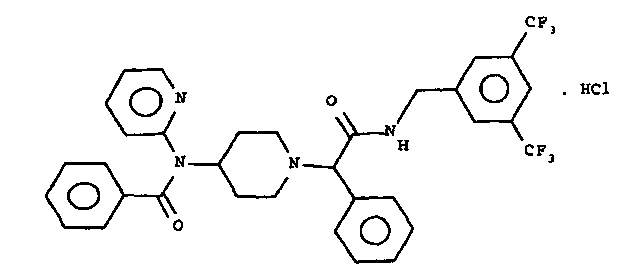

Fp.: 145 -151 °C; FAB-MS: (M+H)⁺ = 641,3

### Beispiel 35:

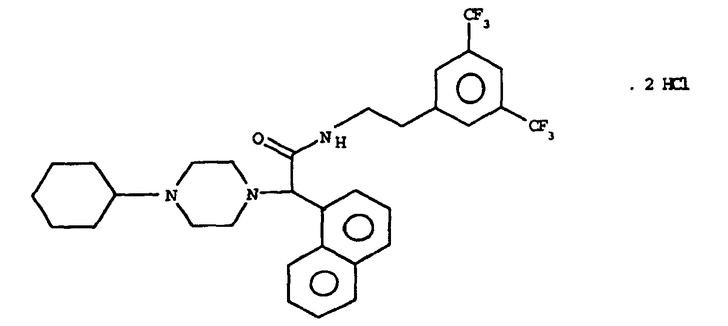

Fp.: 155 - 172°C;
FAB-MS: (M+H)⁺ = 592,2

### Beispiel 36:

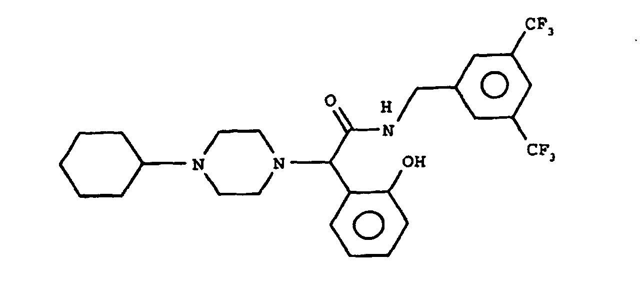

### Beispiel 37:

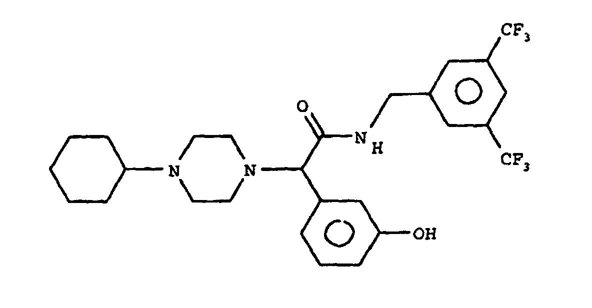

### Beispiel 38:

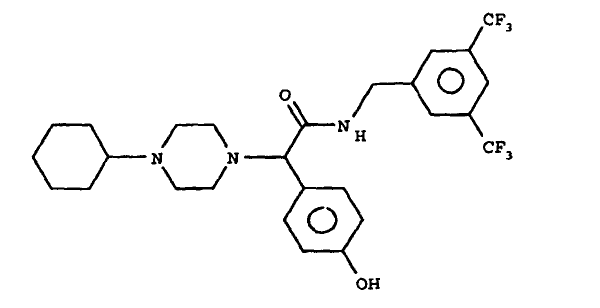

### Beispiel 39:

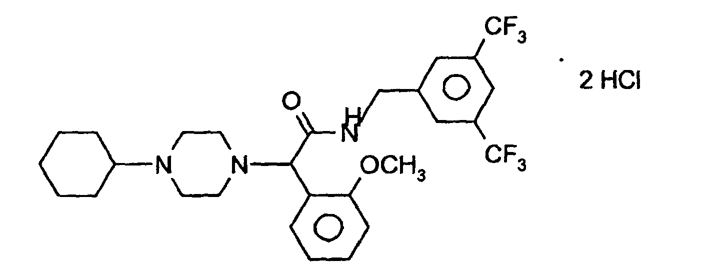

Fp.: 142-150°C.
FAB-MS: (M+H)⁺ = 558,2

### Beispiel 40:

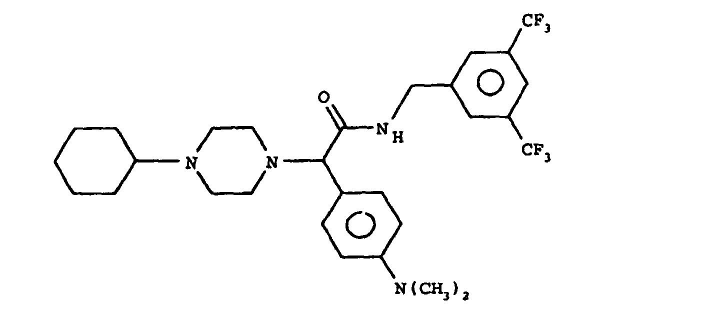

### Beispiel 41:

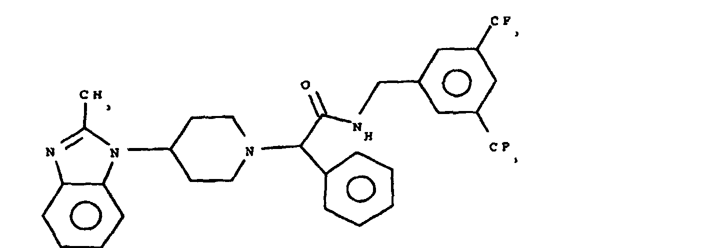

Fp.: 107 - 111°C; FAB-MS: (M+H)⁺ = 575,6

### Beispiel 42:

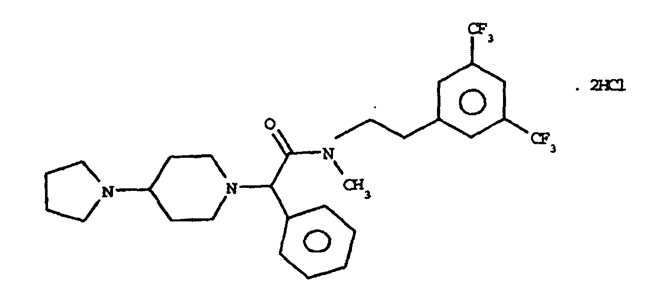

Fp.: >230°C

### Beispiel 43:

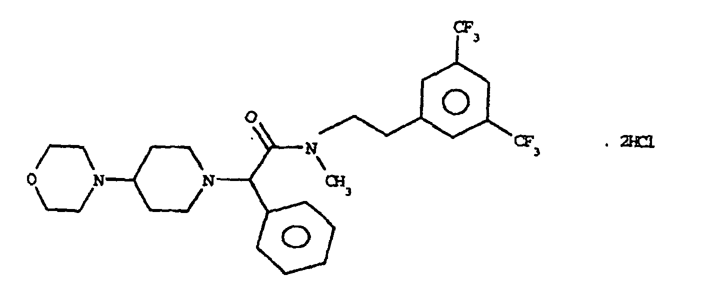

Fp.: >230°C

### Beispiel 44:

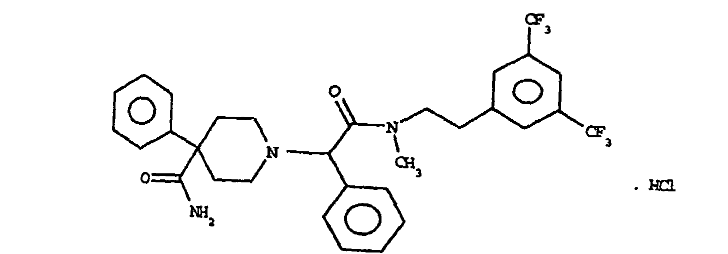

Fp.: 127 - 137°C
FAB-MS: (M+H)⁺ = 592

### Beispiel 45:

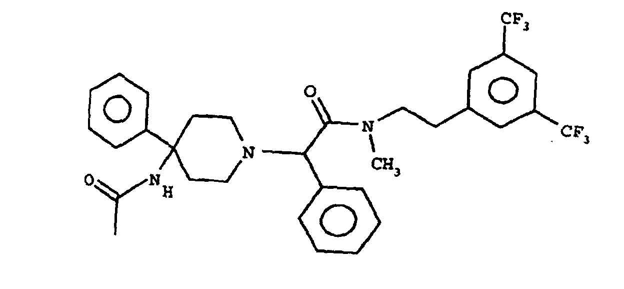

### Beispiel 46:

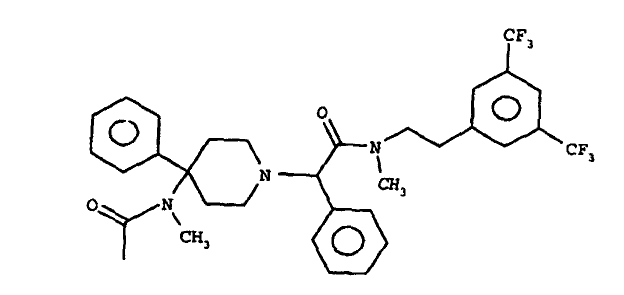

### Beispiel 47:

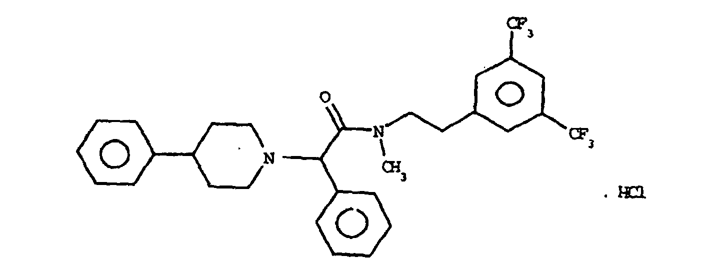

Fp.: 106 - 110°C
FAB-MS: (M+H)⁺ = 549,4

### Beispiel 48:

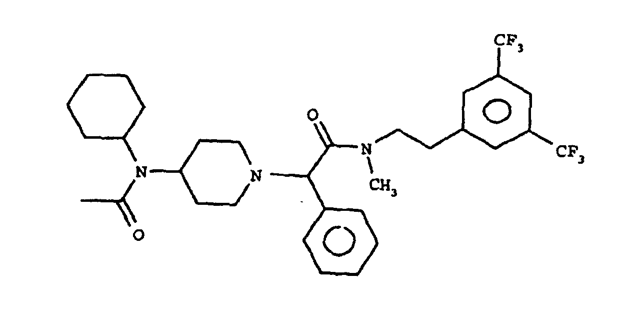

### Beispiel 49:

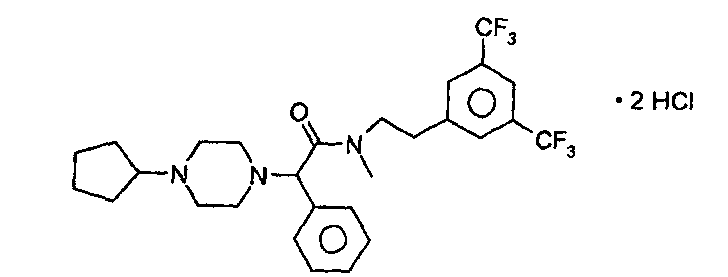

Fp.: 133-143°C
FAB-MS: (M+H)⁺ = 542,3

### Beispiel 50:

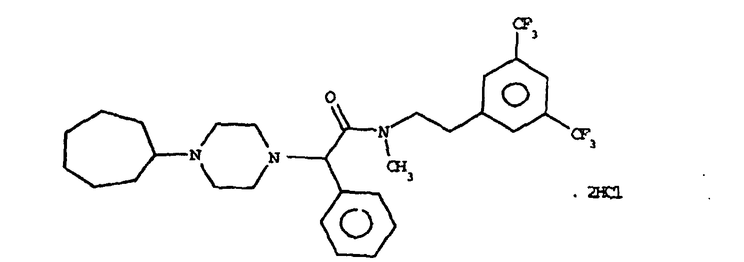

Fp.: 110 - 120°C
FAB-MS: (M+H)⁺ = 570,4

### Beispiel 51:

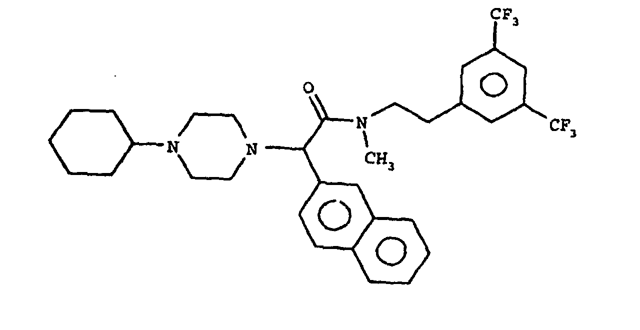

### Beispiel 52:

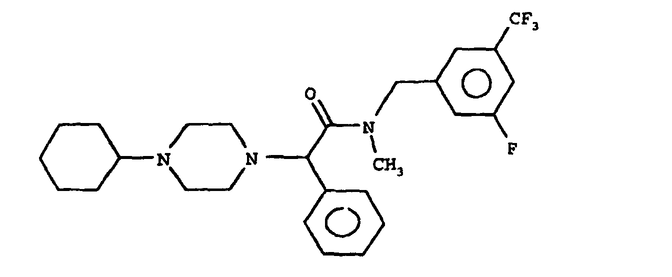

### Beispiel 53:

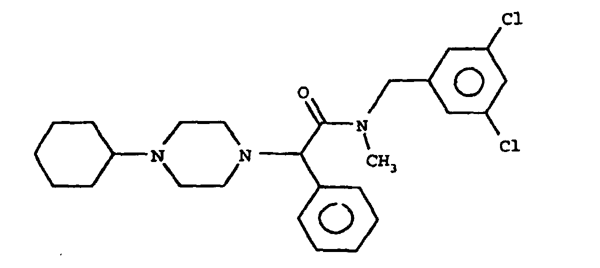

### Beispiel 54:

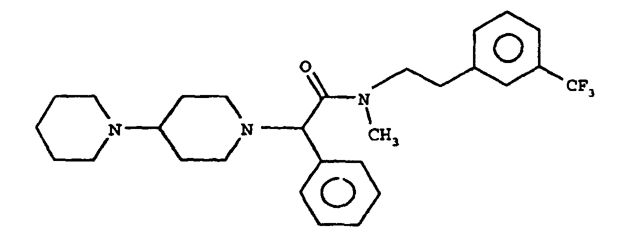

### Beispiel 55

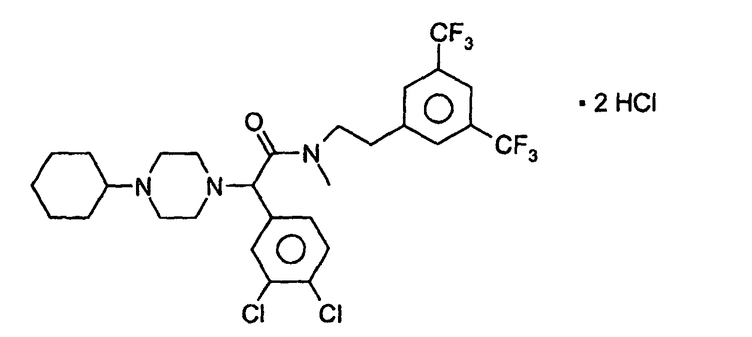

Fp.: 212-216°C (Zers.)
FAB-MS: (M+H)⁺ = 624,3 / 626,3 / 628,3

### Beispiel 56

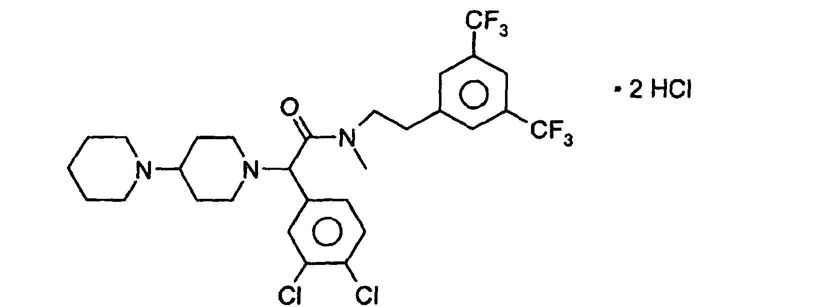

Fp.: 244-246°C (Zers.)
FAB-MS: (M+H)⁺ = 624,1/626,2/628

### Beispiel 57

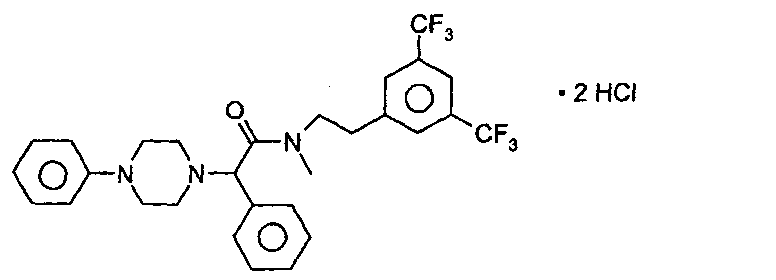

Fp.: 113-123°C.
FAB-MS: (M+H)⁺ = 550,3

### Beispiel 58

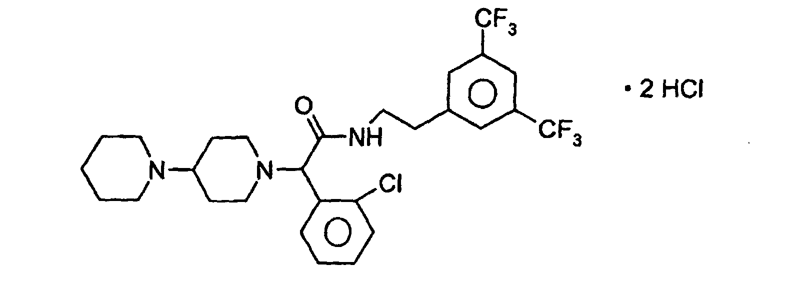

Fp.: 195-205°C.

### Beispiel 59

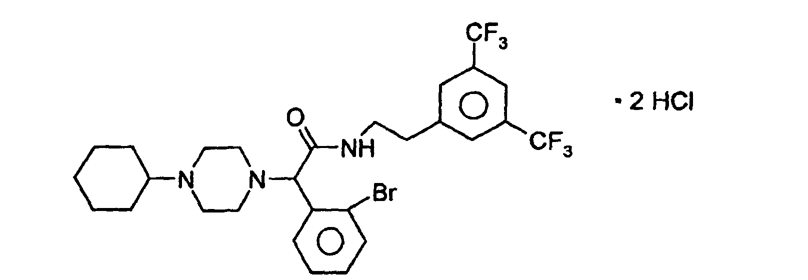

Fp.: 210-218°C.
FAB-MS: (M+H)⁺ = 620/622

### Beispiel 60

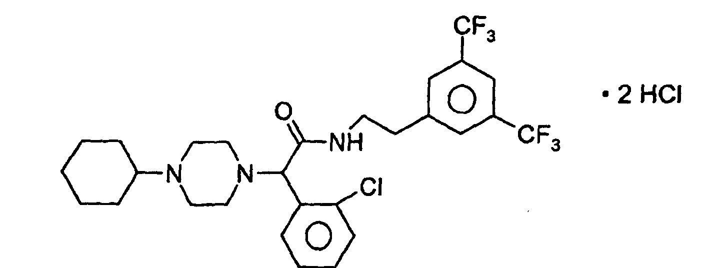

Fp.: 215-224°C
FAB-MS: (M+H)⁺ = 576/578

### Beispiel 61

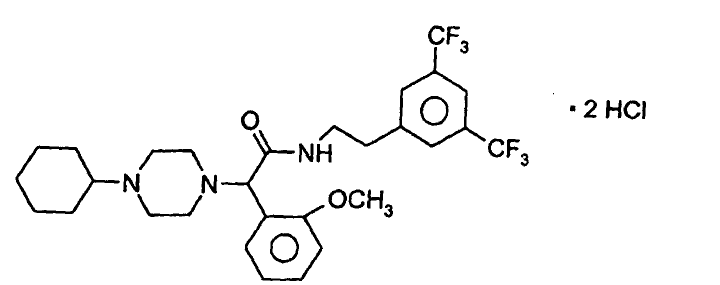

Fp.: 85-92°C
FAB-MS: (M+H)⁺ = 572,5°

### Beispiel 62

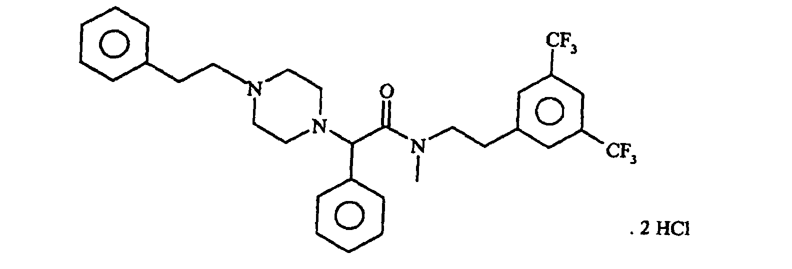

Fp.: 148 - 156 ° C
FAB-MS: (M+H)⁺ = 578,4

### Beispiel 63

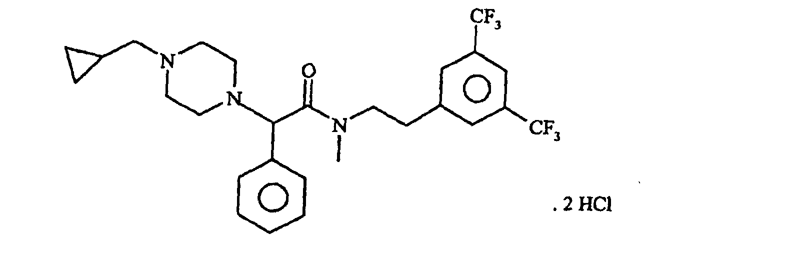

Fp.: 113 - 117 ° C (Zers.)
FAB-MS: (M + H)⁺ = 528,5

### Beispiel 64

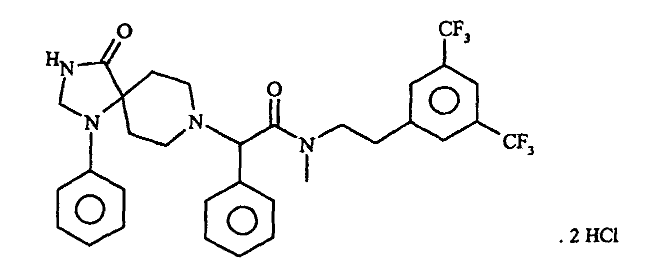

Fp.: 265 - 268 ° C (Zers.)
FAB-MS: (M + H)⁺ = 619,3

### Beispiel 65

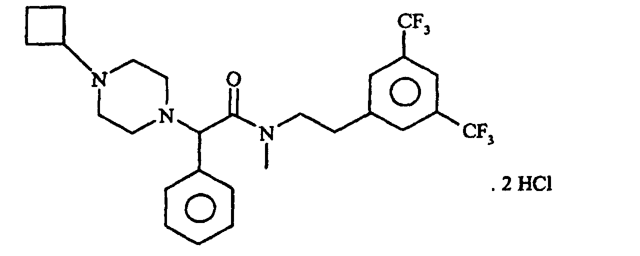

Fp.: 236 - 238 ° C (Zers.)
FAB-MS: (M+H)⁺ = 528,3

### Beispiel 66

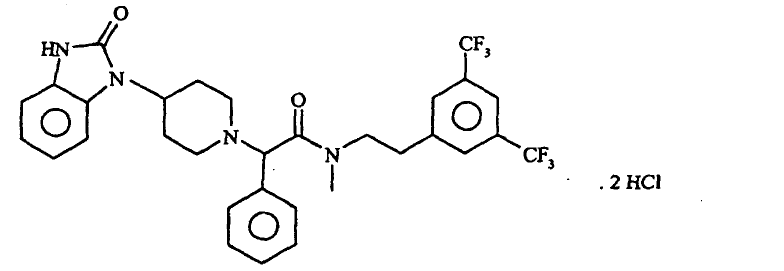

Fp.: 177 - 187 ° C
FAB-MS: (M+H)⁺ = 605,3

### Beispiel 67

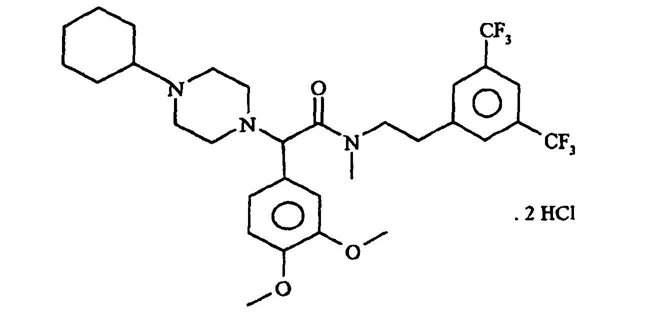

Fp.: 123 - 133°C (Zers.)
FAB-MS: (M + H)⁺ = 616,3

### Beispiel 68

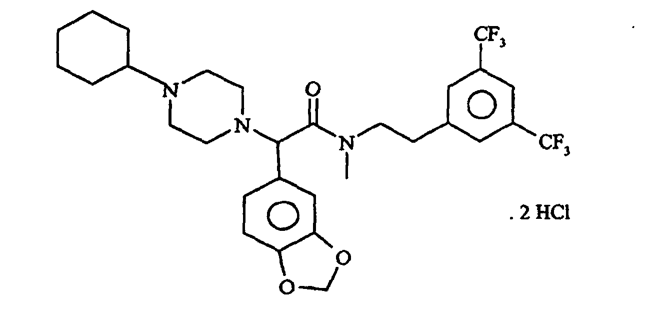

Fp.: 87 - 97 ° C
FAB-MS: (M + H)⁺ = 600,2

### Beispiel 69

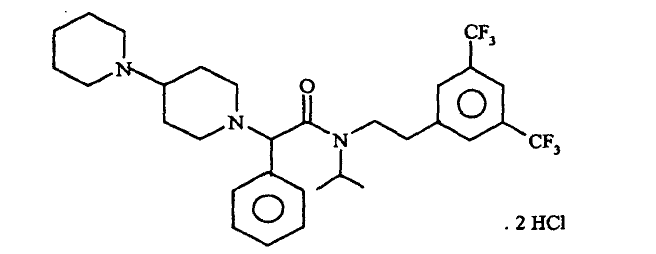

Fp.: > 230 ° C

### Beispiel 70

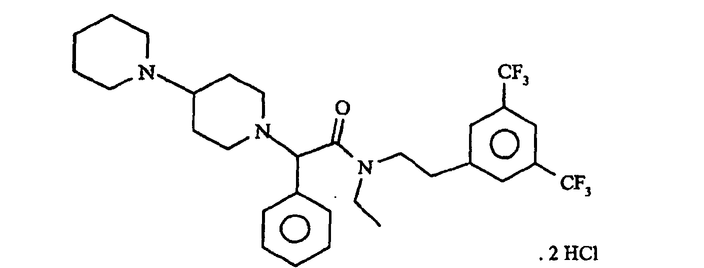

Fp.: > 230 ° C

### Beispiel 71

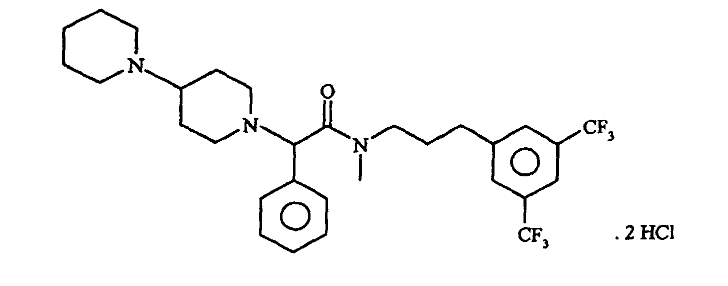

Fp.: > 230 ° C

### Beispiel 72

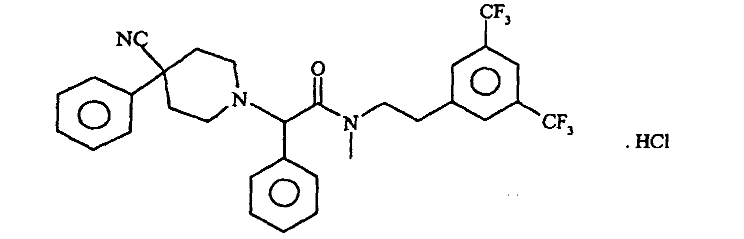

Fp.: 91 - 98 ° C.
FAB-MS: (M+H)⁺ = 574,4

### Beispiel 73

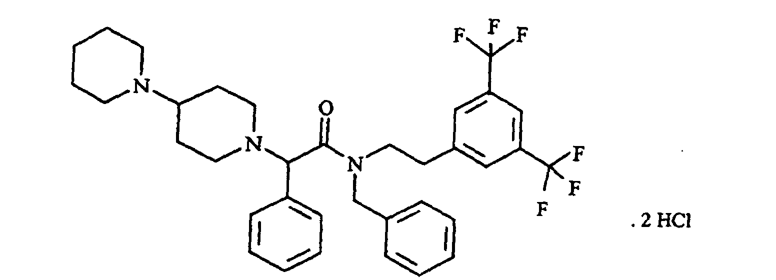

Fp.: 234 - 236 ° C

### Beispiel 74

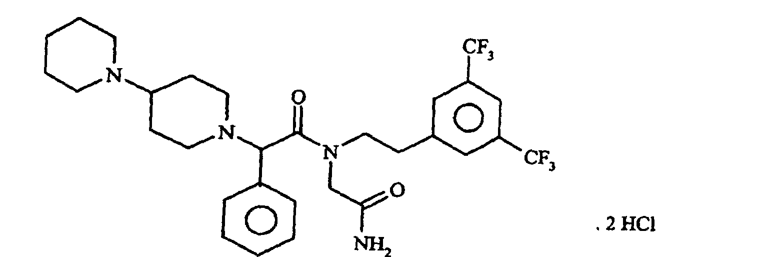

Fp.: 195 - 198 ° C

### Beispiel 75

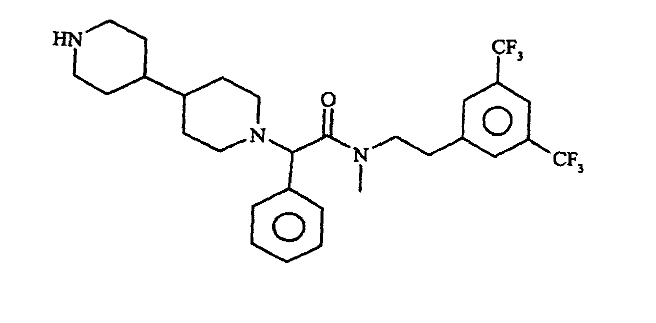

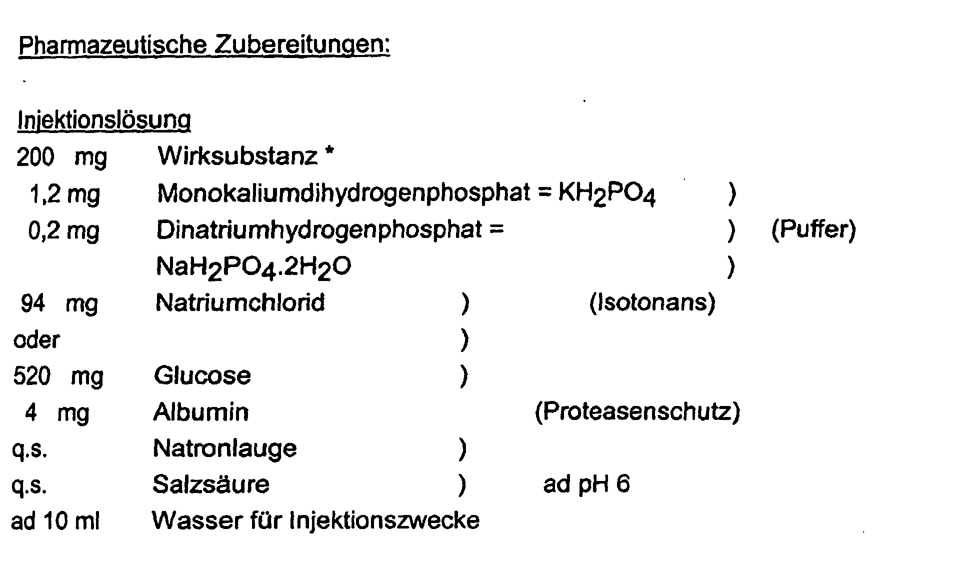

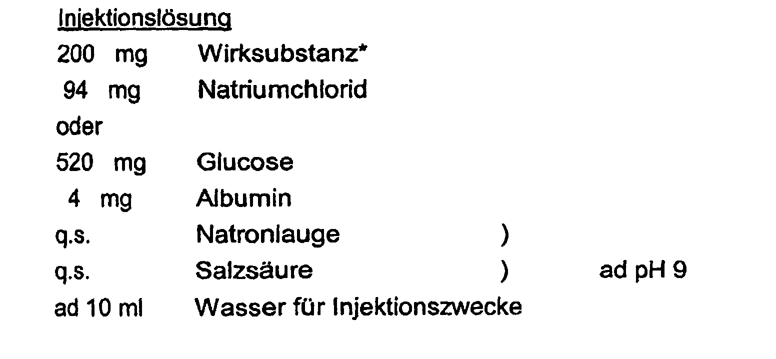

| Lyophilisat | |
|---|---|
| 200 mg | Wirksubstanz* |
| 520 mg | Mannit (Isotonans/Gerüstbildner) |
| 4 mg | Albumin |

| Lösungsmittel 1 für Lyophilisat | |
|---|---|
| 10 ml | Wasser für Injektionszwecke |

| Lösungsmittel 2 für Lyophilisat | |
|---|---|
| 20 mg | Polysorbat®80 = Tween®80 (oberflächenaktiver Stoff) |
| 10 ml | Wasser für Injektionszwecke |

| | |
|---|---|
| * Wirksubstanz: erfindungsgemäße Verbindung, z.B. die der Beispiele 1 bis 78. Dosis für Mensch von 67 kg: 1 bis 500 mg | |

## Patentansprüche

1. Arylglycinamidderivate der allgemeinen Formel I 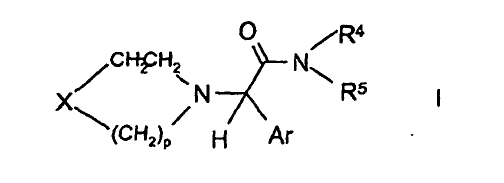 oder deren pharmazeutisch annehmbare Salze,
worin
Ar unsubstituiertes oder 1- oder 2-fach substituiertes Phenyl, oder unsubstituiertes Naphthyl ist, [wobei die Substituenten des Phenyl unabhängig voneinander Halogen (F, Cl, Br, J), OH, Methyl, Methoxy, CF₃, OCF₃ oder Dimethylamin sind] oder Ar durch -OCH₂O-substituiertes Phenyl ist, wobei diese Gruppe die Positionen 2 und 3 oder 3 und 4 des Phenyl verbinden;
p 2 oder 3 ist,
X N(CH₂)ₙR⁶ oder CR⁷R⁸ bedeutet,
worin
n 0, 1 oder 2 ist,
R⁶ (C₃-C₇)Cycloalkyl oder Phenyl ist,
R⁷ und R⁸ eine der folgenden Bedeutungen hat
a) R⁷ ist
Phenyl, Piperidinyl, 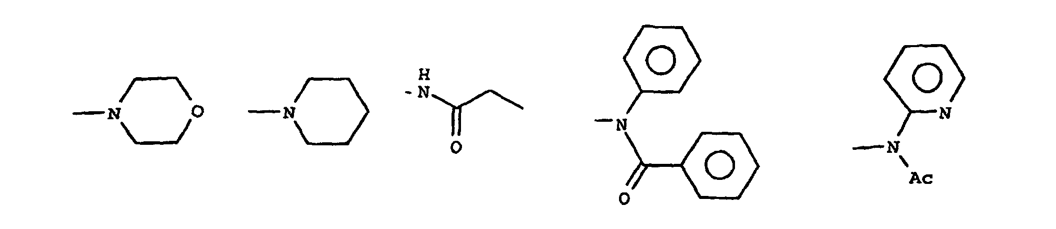 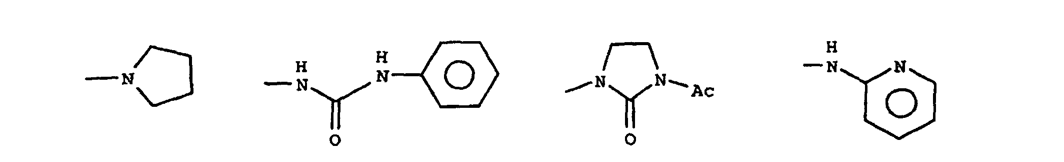 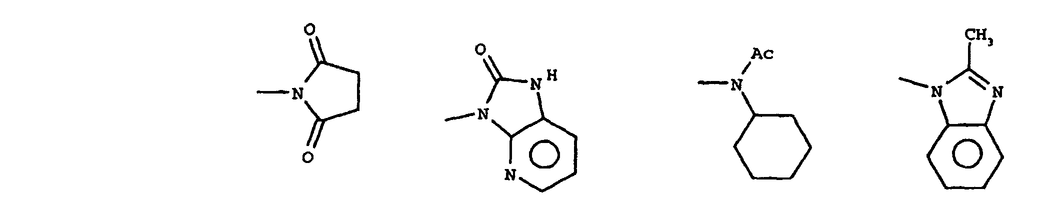 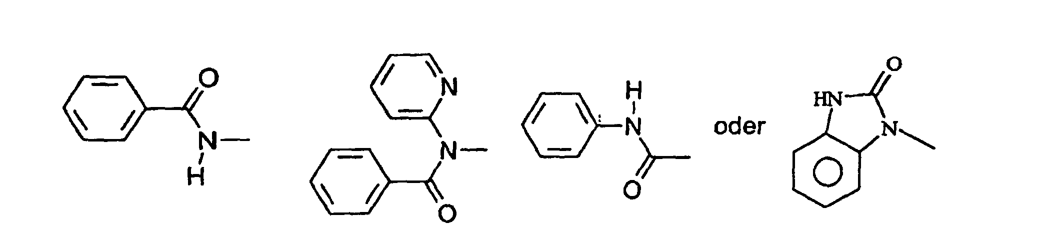 und R⁸ ist H, -CONH₂, -NHC(O)CH₃, -N(CH₃)C(O)CH₃, CN,
oder
c) R⁷ und R⁸ bilden zusammen den Rest 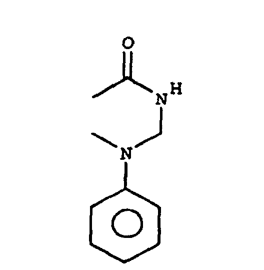
R⁴ Phenyl(C₁-C₄)alkyl oder Naphthyl(C₁-C₄)alkyl bedeutet, worin Phenyl durch 1 bis 3 Substituenten substituiert sein kann, worin die Substituenten unabhängig voneinander Halogen (F, Cl, Br, J), (C₁-C₄)Alkyl, O-(C₁-C₄)Alkyl, CF₃, OCF₃ oder NR¹⁹R²⁰ (worin R¹⁹ und R²⁰ unabhängig voneinander H, Methyl oder Acetyl sind) sind;
und
R⁵ H, (C₁-C₄)Alkyl, (C₃-C₆)Cycloalkyl, CH₂COOH, -CH₂C(O)NH₂, -OH oder Phenyl(C₁-C₄)alkyl bedeutet,
mit der Maßgabe, dass
4-Phenyl-1-(2-phenylessigsäure-N-benzylamid)-piperidin
ausgenommen ist.

2. Verbindung nach Anspruch 1, worin
X N(CH₂)ₙR⁶ ist, worin n und R⁶ wie in Anspruch 1 definiert sind.

3. Verbindung nach Anspruch 2, worin n 0 ist und R⁶ (C₃-C₇)Cycloalkyl.

4. Verbindung nach Anspruch 3, worin R⁶ Cyclobutyl oder Cyclohexyl ist.

5. Verbindung nach Anspruch 1, worin X CR⁷R⁸ ist,worin R⁷und R⁸ wie in Anspruch 1 definiert sind.

6. Verbindung nach Anspruch 5, worin
R⁷ und R⁸ eine der folgenden Bedeutungen hat
a) R⁷ ist
Phenyl, 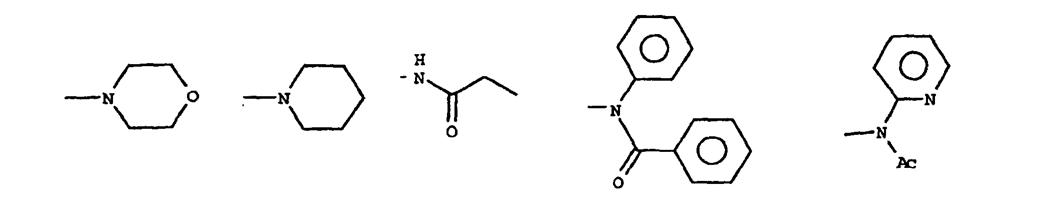 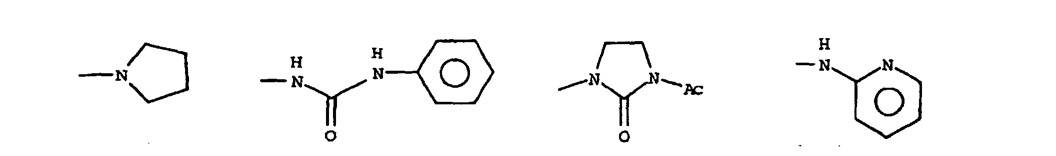 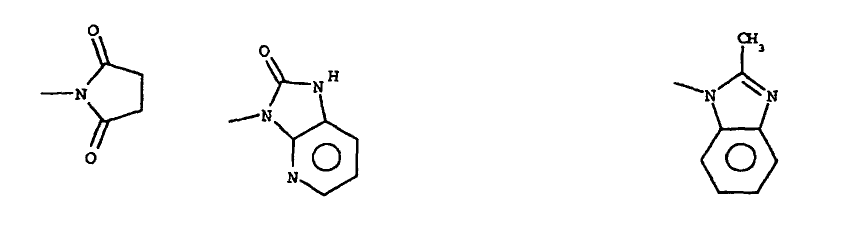 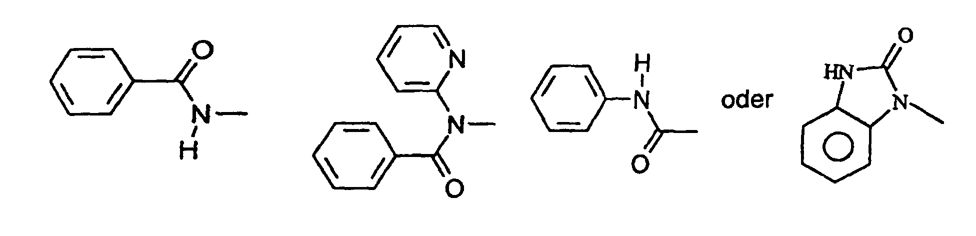 und R⁸ ist H, -CONH₂ oder CN ist,
oder
c) R⁷ und R⁸ bilden zusammen den Rest

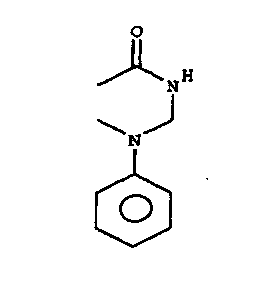

7. Verbindung nach Anspruch 6, worin
R⁷ Phenyl, 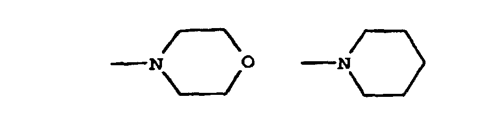 oder 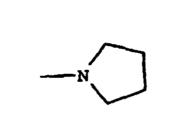 ist
und R⁸ H oder CN ist.

8. Verbindung nach Anspruch 7, worin R⁷ Piperidino und R⁸ H ist.

9. Verbindung nach Anspruch 1, worin
Ar unsubstituiertes oder 1- oder 2-fach substituiertes Phenyl, oder unsubstituiertes Naphthyl ist, [wobei die Substituenten des Phenyl unabhängig voneinander Halogen (F, Cl, Br) Methoxy oder CF₃ sind] oder Ar durch -OCH₂O- substituiertes Phenyl ist, wobei diese Gruppe die Positionen 2 und 3 oder 3 und 4 des Phenyl verbindet.

10. Verbindung nach Anspruch 9, worin Ar Phenyl, 3,4-Dichlorphenyl, 3,4-Dimethoxyphenyl oder 3,4-Methylendioxyphenyl ist.

11. Verbindung nach einem der Ansprüche 1 bis 10, worin
R⁴ Phenyl(C₁-C₃)alkyl bedeutet, worin Phenyl durch 1 oder 2 Substituenten substituiert sein kann, worin die Substituenten unabhängig voneinander Halogen (F, Cl, Br, J), Methyl, Methoxy, CF₃ oder OCF₃ sind;
und
R⁵ H, (C₁-C₃)Alkyl, CH₂COOH, -CH₂C(O)NH₂ oder Phenethyl bedeutet.

12. Verbindung nach Anspruch 11, worin
R⁴ 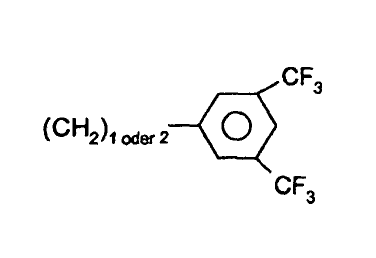 ist
und R⁵ H oder CH₃.

13. Verbindung nach Anspruch 1, die 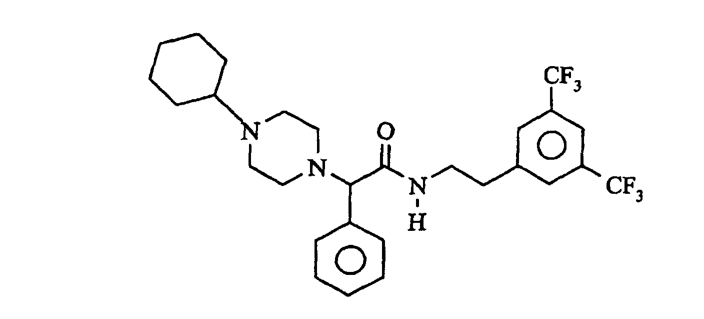 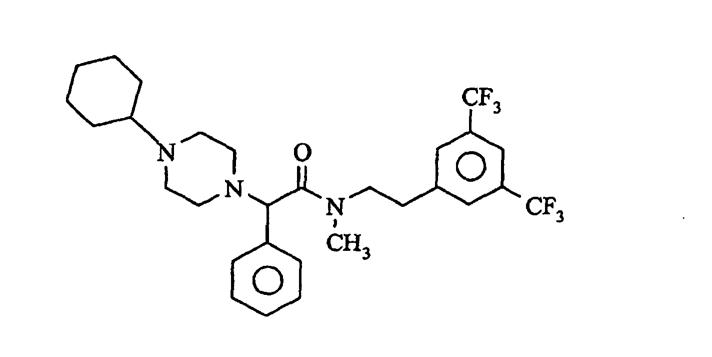 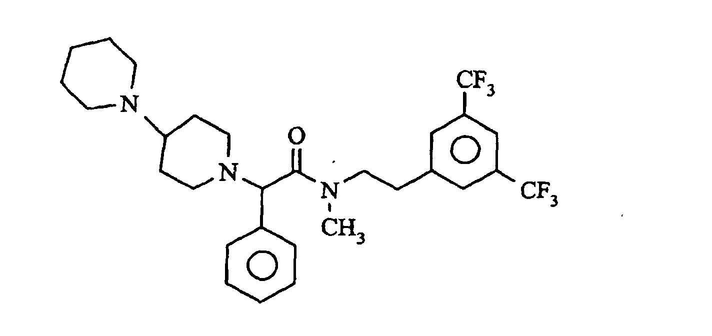 oder 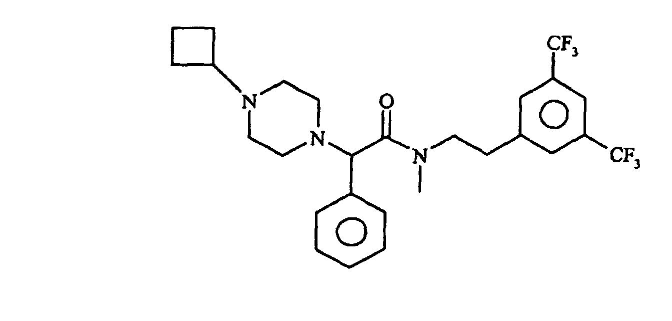 ist.

14. Verbindung nach Anspruch 1, die 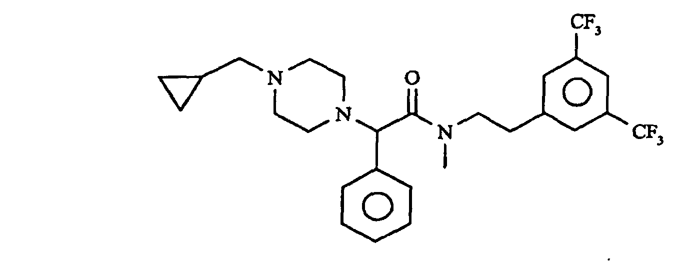 ist.

15. Verbindung nach einem der Ansprüche 1 bis 14, **dadurch gekennzeichnet, dass** die Verbindung als Enantiomer in der (R)- oder (S)-Form vorliegt.

16. Verfahren zur Herstellung einer Verbindung der allgemeinen Formel I nach einem der Ansprüche 1 bis 15, **dadurch gekennzeichnet, daß** man
a) eine Säure 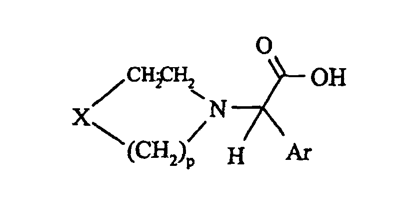 oder deren Halogenid oder Alkylester mit einem Amin 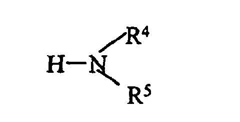 umsetzt;
b) ein α-Halogenarylacetamid 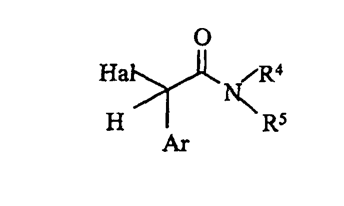 mit einem Amin 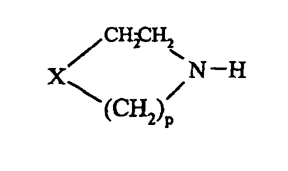 umsetzt; oder
c) eine Verbindung I, in der R⁵ H ist N-alkyliert;
und eine so erhaltene Verbindung als freie Verbindung oder als deren pharmazeutisch annehmbares Salz isoliert.

17. Arylglycinamidderivate der allgemeinen Formel I 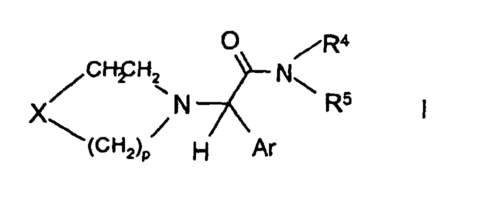 oder deren pharmazeutisch annehmbare Salze, zur therapeutischen Behandlung des menschlichen oder tierischen Körpers,
worin Ar, p, X, R⁴ und R⁵ wie in Anspruch 1 definiert sind.

18. Verbindung nach einem der Ansprüche 1 bis 15 zur therapeutischen Behandlung des menschlichen oder tierischen Körpers.

19. Pharmazeutische Zubereitung enthaltend eine Verbindung nach einem der Ansprüche 1 bis 15.

20. Verwendung einer Verbindung nach einem der Ansprüche 1 bis 15 für die Herstellung einer pharmazeutischen Zubereitung zur Therapie von und zur Vorbeugung gegenüber Neurokinin-vermittelten Krankheiten.

21. Verwendung nach Anspruch 20 zur Therapie von und zur Vorbeugung gegenüber Erkrankungen des Zentralnervensystems, wie Demenz, M. Alzheimer, Schizophrenie, Psychosen, Depression, Kopfschmerzen (z.B. Migräne oder Spannungskopfschmerzen) und Epilepsie.

## Claims

1. Arylglycinamide derivatives of general formula I 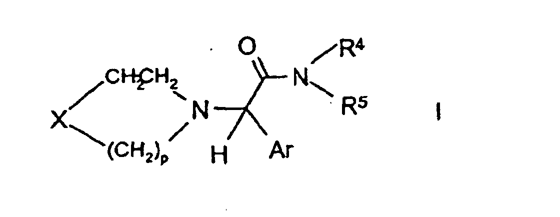 or the pharmaceutically acceptable salts thereof,
wherein
Ar denotes unsubstituted or mono- or disubstituted phenyl, or unsubstituted naphthyl, [in which the substituents of the phenyl independently of each other denote halogen (F, Cl, Br, I), OH, methyl, methoxy, CF₃, OCF₃ or dimethylamine] or Ar is phenyl substituted by -OCH₂O-, this group linking positions 2 and 3 or 3 and 4 of the phenyl;
p is 2 or 3,
X denotes N(CH₂)ₙR⁶ or CR⁷R⁸, wherein
n is 0, 1 or 2,
R⁶ is (C₃₋₇)cycloalkyl or phenyl,
R⁷ and R⁸ have one of the following meanings:
a) R⁷ is phenyl, piperidinyl, 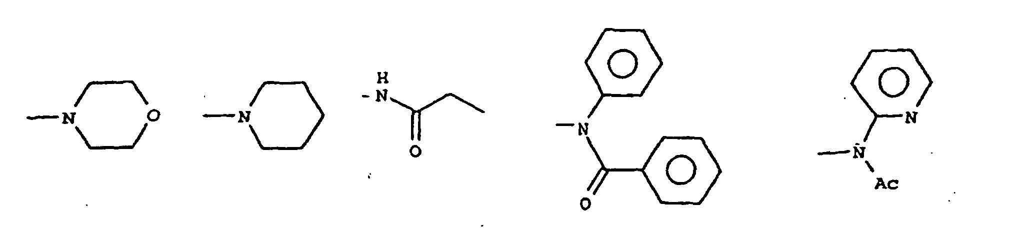 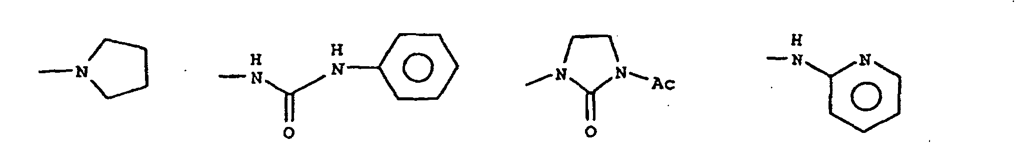 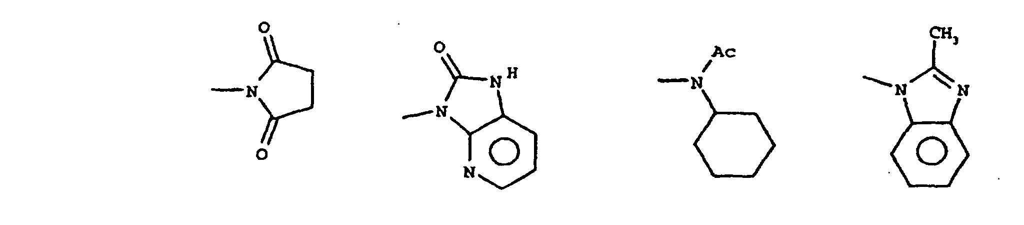 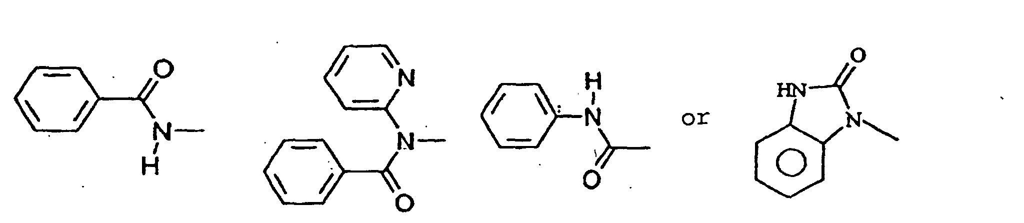 and R⁸ is H, -CONH₂, -NHC(O)CH₃, -N(CH₃)C(O)CH₃, CN
or
c) R⁷ and R⁸ together form the group 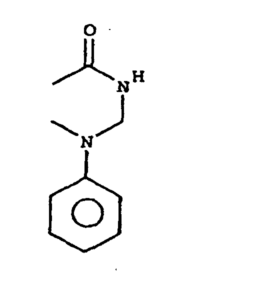
R⁴ denotes phenyl (C₁₋₄) alkyl or naphthyl (C₁₋₄) alkyl, wherein phenyl may be substituted by 1 to 3 substituents, wherein the substituents independently of one another are halogen (F, Cl, Br, I), (C₁₋₄)alkyl, O-(C₁₋₄)alkyl, CF₃, OCF₃ or NR¹⁹R²⁰ (wherein R¹⁹ and R²⁰ independently of one another denote H, methyl or acetyl);
and
R⁵ denotes H, (C₁₋₄)alkyl, (C₃₋₆)cycloalkyl, CH₂COOH, -CH₂C(O)NH₂, -OH or phenyl(C₁₋₄)alkyl,
with the proviso that
4-phenyl-1-(2-phenylacetic acid-N-benzylamide)-piperidine is excepted.

2. Compound according to claim 1, wherein
X denotes N(CH₂)ₙR⁶,
wherein n and R⁶ are defined as in claim 1.

3. Compound according to claim 2, wherein n is 0 and R⁶ is (C₃₋₇) cycloalkyl.

4. Compound according to claim 3, wherein R⁶ is cyclobutyl or cyclohexyl.

5. Compound according to claim 1, wherein X is CR⁷R⁸, wherein R⁷ and R⁸ are defined as in claim 1.

6. Compound according to claim 5, wherein
R⁷ and R⁸ have one of the following meanings:
a)R⁷ is
phenyl, 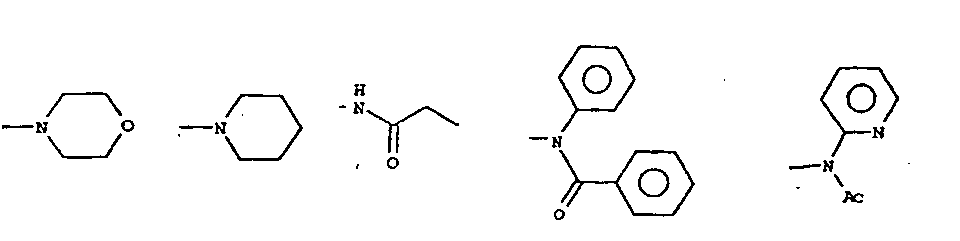 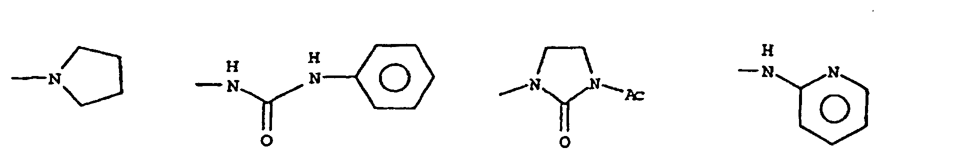 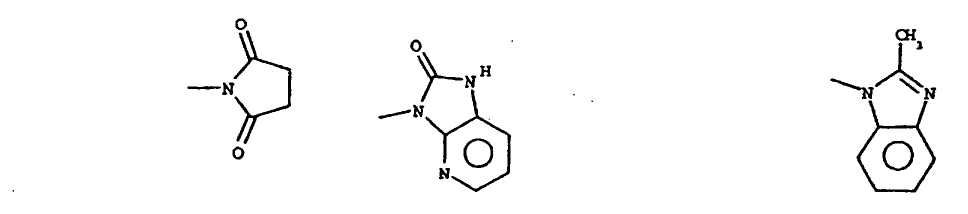 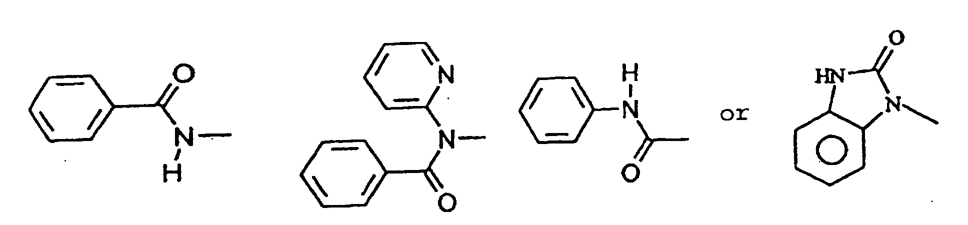 and R⁸ is H, -CONH₂ or CN,
or
c) R⁷ and R⁸ together form the group

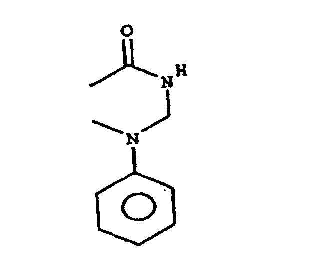

7. Compound according to claim 6, wherein
R⁷ is phenyl, 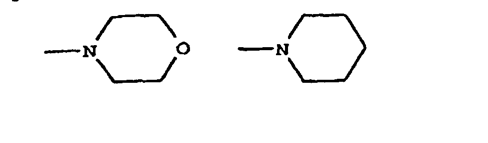 or 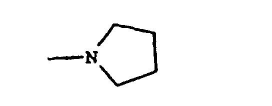
and R⁸ is H or CN.

8. Compound according to claim 7, wherein R⁷ is piperidino and R⁸ is H.

9. Compound according to claim 1, wherein
Ar denotes unsubstituted or mono- or disubstituted phenyl, or unsubstituted naphthyl [wherein the substituents of the phenyl independently of one another are halogen (F, Cl, Br), methoxy or CF₃] or Ar is phenyl substituted by -OCH₂O-, this group connecting positions 2 and 3 or 3 and 4 of the phenyl.

10. Compound according to claim 9, wherein Ar is phenyl, 3,4-dichlorophenyl, 3,4-dimethoxyphenyl or 3,4-methylenedioxyphenyl.

11. Compound according to one of claims 1 to 10, wherein
R⁴ denotes phenyl(C₁₋₃)alkyl, wherein phenyl may be substituted by 1 or 2 substituents, the substituents independently of one another being halogen (F, Cl, Br, I), methyl, methoxy, CF₃ or OCF₃;
and
R⁵ denotes H, (C₁₋₃)alkyl, CH₂COOH, -CH₂C(O)NH₂ or phenethyl.

12. Compound according to claim 11, wherein
R⁴ is 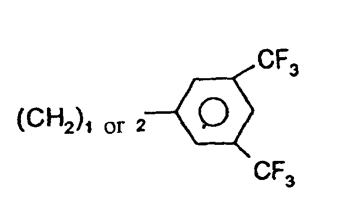 and R⁵ is H or CH₃.

13. Compound according to claim 1 which is 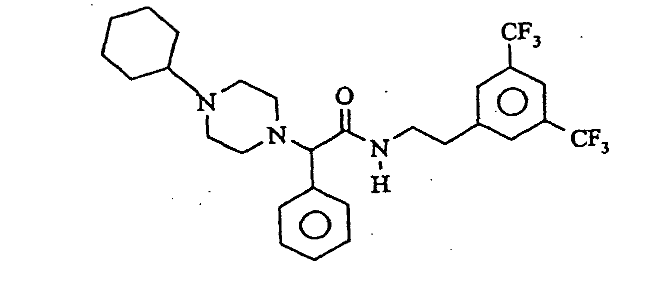 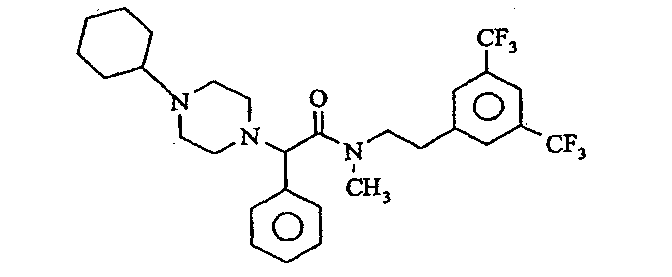 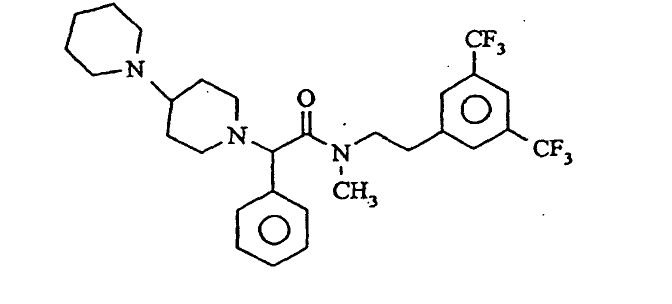 or

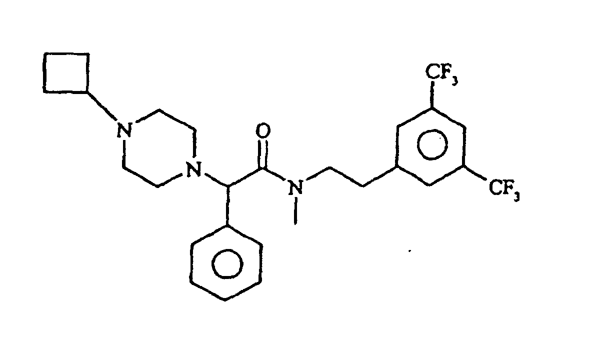

14. Compound according to claim 1 which is

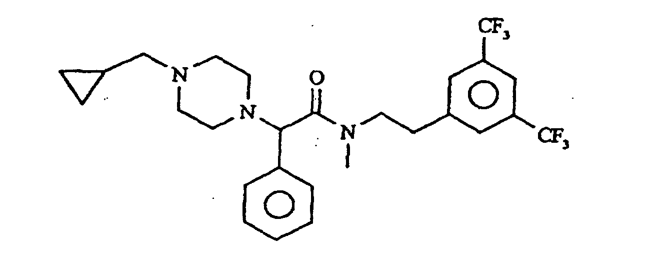

15. Compound according to one of claims 1 to 14, . **characterised in that** the compound is present as the enantiomer in the (R) or (S) form.

16. Process for preparing a compound of general formula I according to one of claims 1 to 15, **characterised in that**
a) an acid 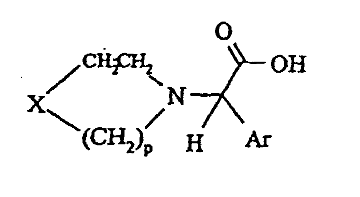 or a halide or alkylester thereof is reacted with an amine 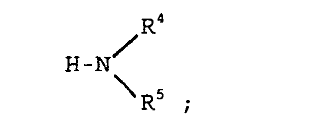
b) an α-haloarylacetamide
is reacted with an amine or 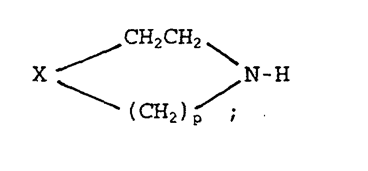
c) a compound I wherein R⁵ is H is N-alkylated;
and a compound thus obtained is isolated as a free compound or as a pharmaceutically acceptable salt thereof.

17. Arylglycinamide derivatives of general formula I 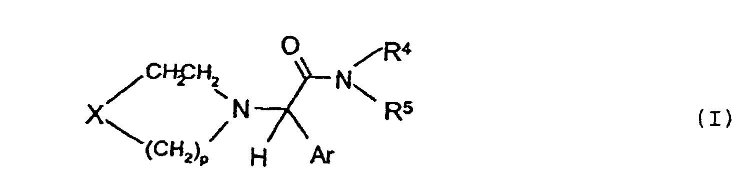 or the pharmaceutically acceptable salts thereof, for the therapeutic treatment of the human or animal body, wherein Ar, p, X, R⁴ and R⁵ are defined as in claim 1.

18. Compound according to one of claims 1 to 15 for the therapeutic treatment of the human or animal body.

19. Pharmaceutical preparation containing a compound according to one of claims 1 to 15.

20. Use of a compound according to one of claims 1 to 15, for preparing a pharmaceutical preparation for the treatment and prevention of neurokinin-mediated diseases.

21. Use according to claim 20 for the treatment and prevention of diseases of the central nervous system such as dementia, Alzheimer's disease, schizophrenia, psychosis, depression, headaches (e.g. migraine or tension headaches) and epilepsy.

## Revendications

1. Dérivés d'arylglycinamide de formule générale I 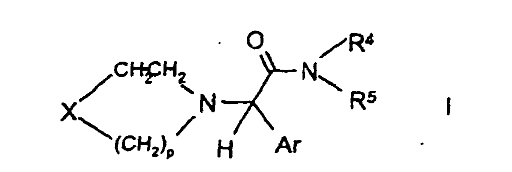 ou leurs sels pharmaceutiquement acceptables,
où
Ar est phényle non substitué ou substitué 1 ou 2 fois ou naphtyle non substitué [où les substituants du phényle sont indépendamment l'un de l'autre halogène (F, Cl, Br, I), OH, méthyle, méthoxy, CF₃, OCF₃ ou diméthylamine] ou Ar est phényle substitué par -OCH₂O-, où ce groupe relie les positions 2 et 3 ou 3 et 4 du phényle ;
p est 2 ou 3,
X signifie N(CH₂)ₙR⁶ ou CR⁷R⁸,
où
n est 0, 1 ou 2,
R⁶ est (C₃-C₇)cycloalkyle ou phényle,
R⁷ et R⁸ ont l'une des significations suivantes
a) R⁷ est
phényle, pipéridinyle, 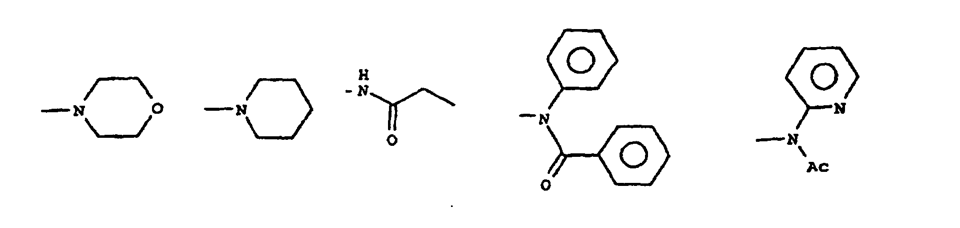 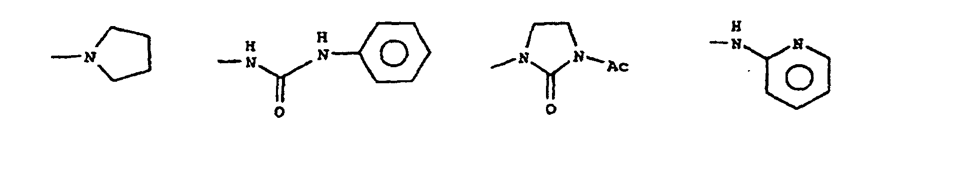 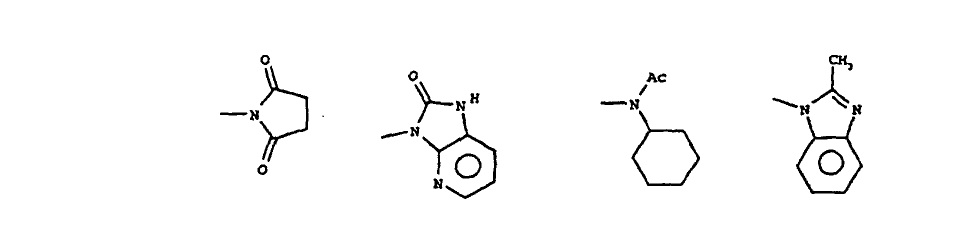 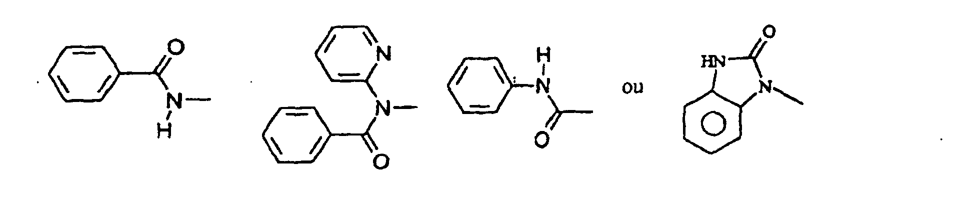 et R⁸ est H, -CONH₂, -NHC(O)CH₃, -N(CH₃)C(O)CH₃, CN,
ou
c) R⁷ et R⁸ forment ensemble le reste 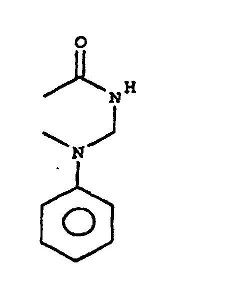
R⁴ signifie phényl(C₁-C₄)alkyle ou naphtyl(C₁-C₄)alkyle, où phényle peut être substitué par 1 à 3 substituants, où les substituants sont indépendamment les uns des autres halogène (F, Cl, Br, I), (C₁-C₄)alkyle, O-(C₁-C₄)alkyle, CF₃, OCF₃ ou NR¹⁹R²⁰ (où R¹⁹ et R²⁰ sont indépendamment l'un de l'autre H, méthyle ou acétyle) ;
et
R⁵ signifie H, (C₁-C₄)alkyle, (C₃-C₆)cycloalkyle, CH₂COOH, -CH₂C(O)NH₂, -OH ou phényl(C₁-C₄)alkyle,
avec la conditions que
la 4-phényl-1-(2-phénylacéto-N-benzylamido)-pipéridine soit exclue.

2. Composé selon la revendication 1 où
X est N(CH₂)ₙR⁶ où n et R⁶ sont définis comme dans la revendication 1.

3. Composé selon la revendication 2 où n est 0 et R⁶ est (C₃-C₇)cycloalkyle.

4. Composé selon la revendication 3 où R⁶ est cyclobutyle ou cyclohexyle.

5. Composé selon la revendication 1 où X est CR⁷R⁸ où R⁷ et R⁸ sont définis comme dans la revendication 1.

6. Composé selon la revendication 5 où
R⁷ et R⁸ ont l'une des significations suivantes
a) R⁷ est
phényle, 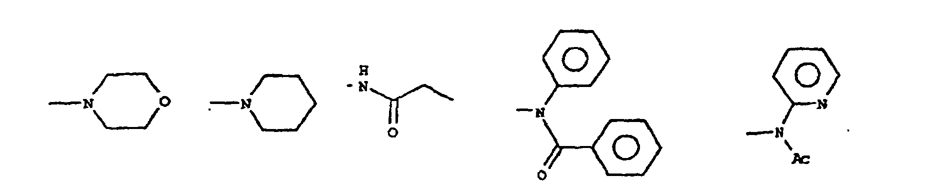 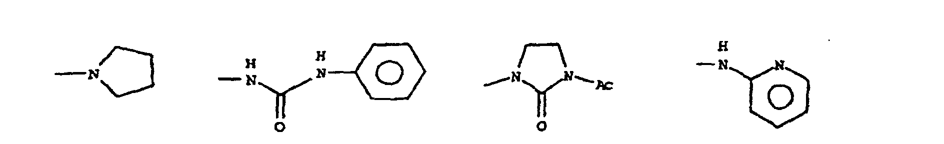 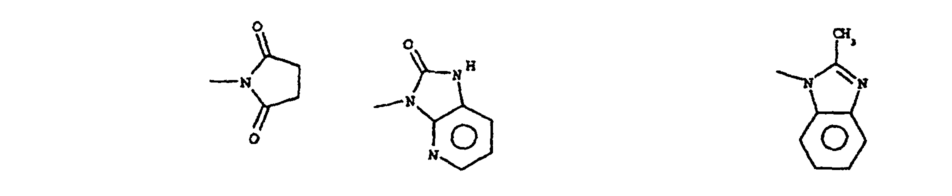 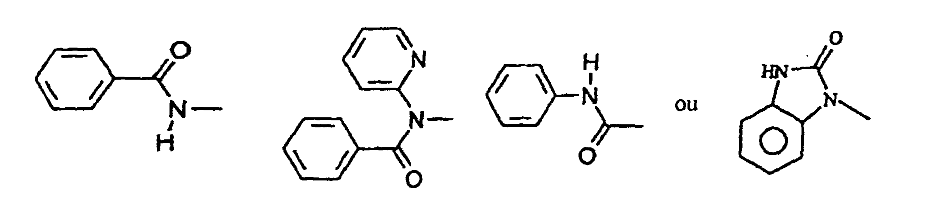 et R⁸ est H, -CONH₂ ou CN,
ou
c) R⁷ et R⁸ forment ensemble le groupement

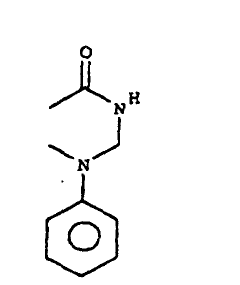

7. Composé selon la revendication 6 où
R⁷ est phényle 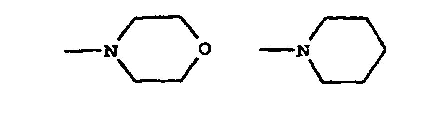 ou 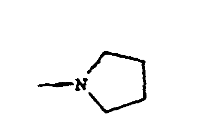 et R⁸ est H ou CN.

8. Composé selon la revendication 7 où R⁷ est pipéridino et R⁸ est H.

9. Composé selon la revendication 1 où
Ar est phényle non substitué ou substitué 1 ou 2 fois ou naphtyle non substitué [où les substituants du phényle sont indépendamment l'un de l'autre halogène (F, Cl, Br), méthoxy ou CF₃] ou Ar est phényle substitué par -OCH₂O-, où ce groupe relie les positions 2 et 3 ou 3 et 4 du phényle.

10. Composé selon la revendication 9 où Ar est phényle, 3,4-dichlorophényle, 3,4-diméthoxyphényle ou 3,4-méthylènedioxyphényle.

11. Composé selon l'une des revendications 1 à 10 où
R⁴ signifie phényl(C₁-C₃)alkyle, où phényle peut être substitué par 1 ou 2 substituants, où les substituants sont indépendamment l'un de l'autre halogène (F, Cl, Br, I), méthyle, méthoxy, CF₃ ou OCF₃ ;
et
R⁵ signifie H, (C₁-C₃)alkyle, CH₂COOH, -CH₂C(O)NH₂ ou phénéthyle.

12. Composé selon la revendication 11 où
R⁴ est 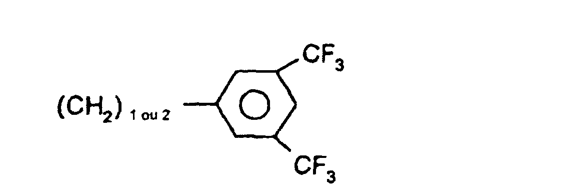 et R⁵ est H ou CH₃.

13. Composé selon la revendication 1 qui est 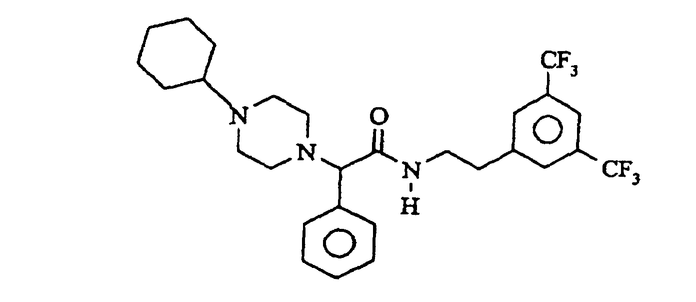 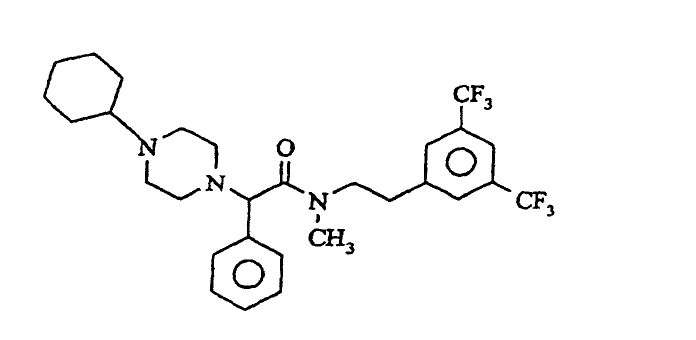 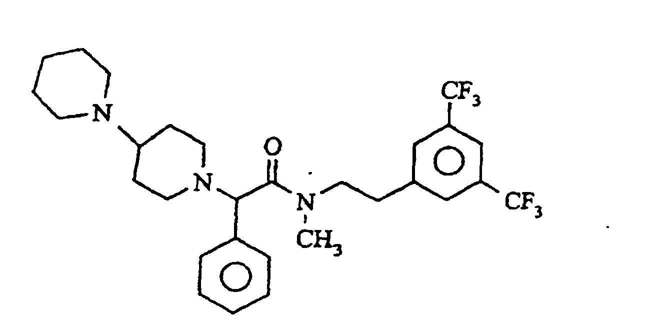 ou

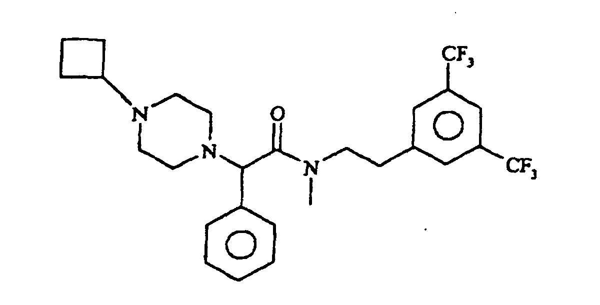

14. Composé selon la revendication 1 qui est

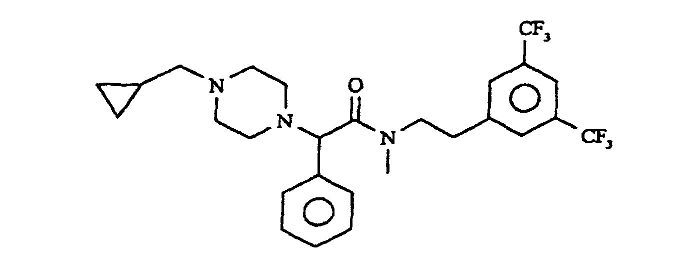

15. Composé selon l'une des revendications 1 à 14 **caractérisé en ce que** le composé est présent sous forme d'énantiomère sous la forme (R) ou (S).

16. Procédé de préparation d'un composé de formule générale I selon l'une des revendications 1 à 15 **caractérisé en ce que**
a) on fait réagir un acide 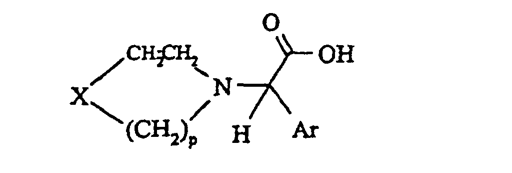 ou son halogénure ou alkylester avec une amine 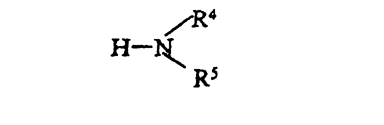
b) on fait réagir un α-halogénoarylacétamide 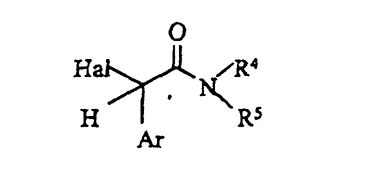 avec une amine 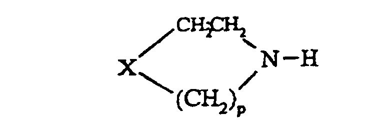 ou bien
c) on N-alkyle un composé I où R⁵ est H ;
et on isole un composé ainsi obtenu sous forme de composé libre ou sous forme de son sel pharmaceutiquement acceptable.

17. Dérivés d'arylglycinamide de formule générale 1 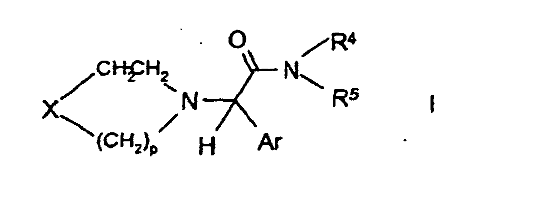 ou leurs sels pharmaceutiquement acceptables, pour le traitement thérapeutique du corps humain ou animal,
où Ar, p, X, R⁴ et R⁵ sont définis comme dans la revendication 1.

18. Composé selon l'une des revendications 1 à 15 pour le traitement thérapeutique du corps humain ou animal.

19. Préparation pharmaceutique contenant un composé selon l'une des revendications 1 à 15.

20. Utilisation d'un composé selon l'une des revendications 1 à 15 pour la production d'une préparation pharmaceutique pour la thérapie ou la prévention des maladies dues à des neurokinines.

21. Utilisation selon la revendication 20 pour la thérapie et la prévention des maladies du système nerveux central, comme la démence, la maladie d'Alzheimer, la schizophrénie, les psychoses, la dépression, les céphalées (par exemple migraine ou céphalée par tension nerveuse) et l'épilepsie.
